# EUROPÄISCHE PATENTSCHRIFT

(11) **EP 2 326 639 B1**
(45) Veröffentlichungstag und Bekanntmachung des Hinweises auf die Patenterteilung: **19.08.2015**
(21) Anmeldenummer: 09777315.4
(22) Anmeldetag: 21.07.2009
(51) Int. Cl.: C07D 401/12, C07D 401/14, A61K 31/4545, A61K 31/496, A61P 25/28

(54) **SUBSTITUIERTE PYRIDINE UND IHRE VERWENDUNG ALS GSK3 INHIBITOREN**
SUBSTITUTED PYRIDINES AND ITS USE AS GSK3 INHIBITORS
PYRIDINES SUBSTITUEES COMME INHIBITEURS DE GSK3

(30) Priorität: 30.07.2008 DE 102008035552
(43) Veröffentlichungstag der Anmeldung: 01.06.2011
(73) Patentinhaber: Bayer Intellectual Property GmbH, 40789 Monheim (DE)
(72) Erfinder: SIEGEL, Stephan, 10779 Berlin (DE); WILMEN, Andreas, 50676 Köln (DE); SVENSTRUP, Niels, 42553 Velbert (DE); GNOTH, Mark, Jean, 40822 Mettmann (DE); TERSTEEGEN, Adrian, 42111 Wuppertal (DE); RESTER, Ulrich, 42115 Wuppertal (DE)
(74) Vertreter: BIP Patents
(86) Internationale Anmeldenummer: PCT/EP2009/005266
(87) Internationale Veröffentlichungsnummer: WO 2010/012398

(56) Entgegenhaltungen:
- WO-A1-2009/035684
- WO-A2-02/20495

## Beschreibung

Die Erfindung betrifft substituierte Pyridine und Verfahren zu ihrer Herstellung sowie ihre Verwendung zur Herstellung von Arzneimitteln zur Behandlung und/oder Prophylaxe von Krankheiten, insbesondere von hämatologischen Erkrankungen, vorzugsweise von Leukopenien und Neutropenien.

Glykogen Synthase Kinase 3 (GSK3) gehört zur Familie der Serine/Threonin-Kinasen. Spezifische Substrate sind unter anderem Zytoskelettproteine und Transkriptionsfaktoren. Zwei Isoformen, GSK3α und GSK3β, wurden bisher identifiziert (Woodgett JR., Trends Biochem. Sci. (1991), 16(5), 177-81). Beide Isoformen sind in vornehmlich ruhenden, nicht proliferierenden Zellen konstitutiv aktiv.

GSK3β kommt eine zentrale Bedeutung innerhalb des Wnt/Wingless-Signaltransduktionsweges zu. Dieser stellt einer der wichtigsten, evolutionärkonservierten Signalsysteme dar. Wnt-Signale kontrollieren sehr frühe musterbildende Prozesse während der Embryogenese, sie induzieren Mesodermbildung und viele Organe, und sie steuern die Proliferation und Differenzierung von Stammzellen (Wodarz A., Nusse R., Annu. Rev. Cell Dev. Biol. (1998), 14, 59-88; Kirstetter et al., Nat Immunol. (2006), 7(10), 1048-56). Der Wnt-Signalweg ist intrazellulär aufgegliedert, wodurch unterschiedlichste Prozesse gesteuert werden können. Innerhalb der Wnt-Kaskade ist die Glykogen Synthase Kinase 3 Bestandteil eines Multiproteinkomplexes, zu dem u.a. das Strukturmoleküle Axin, das Tumorsuppressor-Protein APC sowie der Transkriptionskofaktor β-Catenin gehören. β-Catenin ist dabei das wichtigste Substrat der GSK3β. Die Konsequenz dieser GSK3β-vermittelten Phophorylierung ist der proteasomale Abbau von β-Catenin. Inhibition der GSK3-Aktivität führt zu einer Akkumulation des β-Catenins in der Zelle mit einer sich anschließenden Translokation in den Zellkern. Dort fungiert β-Catenin als ein Kofaktor in Transkriptionskomplexe und damit für die Expression definierter Zielgene mit verantwortlich.

Strahlen- oder Chemotherapien gehören zu den Standardansätzen bei der Krebsbekämpfung. Beide Therapieformen sind im Bezug auf ihre Zielzellen unspezifisch, d.h. es werden nicht nur Tumorsondern auch nicht-transformierte, proliferierende Zellen getroffen. Zu diesen nichttransformierten, proliferienden Zellen gehören auch hämatopoetische Vorläuferzellen, die sich u.a. zu neutrophilen Granulozyten entwickeln. Eine signifikante Verringerung der Anzahl an Neutrophilen wird als Neutropenie bezeichnet. Eine durch Chemo- oder Strahlentherapie induzierte Neutropenie resultiert klinisch in einer erhöhten Infektanfälligkeit. Bei ausgeprägter Neutropenie erhöht sich die Morbidität und unter Umständen auch die Mortalität einer Therapie (O'Brien et al., British Journal of Cancer (2006), 95, 1632 - 1636).

Inhibition der GSK3-Aktivität führt zu einer gesteigerten Proliferations- und Differenzierungsrate hämatopoetischer Stammzellen und kann dementsprechend zur therapeutischen Intervention hinsichtlich einer therapieinduzierten Neutropenie genutzt werden.

WO 99/65897 und WO 02/20495 beschreiben unter anderem Pyridine als Glykogen Synthase Kinase 3 (GSK3) Inhibitoren zur Behandlung von Diabetes, Krebs und Erkrankungen des zentralen Nervensystems. WO 2003/049739 beschreibt Pyrimidine als Glykogen Synthase Kinase 3 (GSK3) Inhibitoren zur Behandlung von Diabetes, Krebs und Erkrankungen des zentralen Nervensystems.

Eine Aufgabe der vorliegenden Erfindung ist daher die Bereitstellung neuer Verbindungen als GSK3β-Inhibitoren zur Behandlung von hämatologischen Erkrankungen, vorzugsweise von Neutropenie bei Menschen und Tieren.

Gegenstand der Erfindung sind Verbindungen der Formel 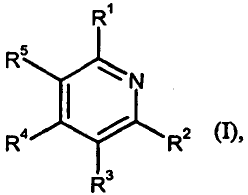 in welcher
R¹ für eine Gruppe der Formel 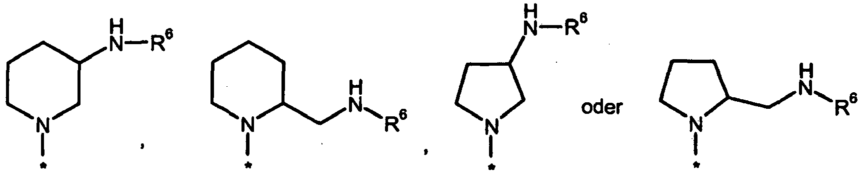 steht,
   wobei
   * die Anknüpfstelle an den Heterocyclus bedeutet,
   R⁶ für Pyrid-2-yl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,2-Pyrazol-5-yl steht,
   wobei Pyrid-2-yl, Pyrimid-2-yl, 1,3-Thiazol-2-yl und 1,3-Thiazol-4-yl substituiert sind mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino), C₁-C₄-Alkylcarbonyl, C₁-C₄-Alkoxycarbonyl, C₁-C₄-Alkylaminocarbonyl und C₃-C₆-Cycloalkylcarbonyl,
   worin Alkyl, Alkylamino, Alkylcarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
   und
   wobei 2-Aminopyrimid-4-yl, 2-Cyclopropylaminopyrimid-4-yl, 2-Methylamino-pyrimid-4-yl, 2-Ethylaminopyrimid-4-yl und 1,2-Pyrazol-5-yl substituiert sein können mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
R² für Phenyl steht,
   wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
   worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
R³ für Wasserstoff, Halogen, Cyano, Trifluormethyl, C₁-C₃-Alkyl oder Cyclopropyl steht,
   entweder
R⁴ für Wasserstoff, Halogen, Cyano, Trifluormethyl, C₁-C₃-Alkyl oder Cyclopropyl steht,
   und
R⁵ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
   wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   und
   wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
   worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   oder
R⁴ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
   wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   und
   wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
   worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   und
R⁵ für Wasserstoff, Halogen, Cyano, Trifluormethyl, C₁-C₃-Alkyl oder Cyclopropyl steht,
   und ihre Salze, ihre Solvate und die Solvate ihrer Salze.

Erfindungsgemäße Verbindungen sind die Verbindungen der Formel (I) und deren Salze, Solvate und Solvate der Salze, sowie die von Formel (I) umfassten, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin, *N*-Methylpiperidin und Cholin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Außerdem umfasst die vorliegende Offenbarung auch Prodrugs der erfindungsgemäßen Verbindungen. Der Begriff "Prodrugs" umfaßt Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl per se und "Alk" und "Alkyl" in Alkoxy, Alkylamino, Alkylcarbonyl, Alkoxycarbonyl, Alkylaminocarbonyl, Alkylcarbonylamino, Alkylsulfonyl, Alkylsulfonylamino und Alkylaminosulfonyl stehen für einen linearen oder verzweigten Alkylrest mit 1 bis 4 Kohlenstoffatomen, beispielhaft und vorzugsweise für Methyl, Ethyl, n-Propyl, iso-Propyl, n-Butyl und tert-Butyl.
Alkoxy steht beispielhaft und vorzugsweise für Methoxy, Ethoxy, n-Propoxy, iso-Propoxy, n-Butoxy und tert-Butoxy.
Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, iso-Propylamino, tert-Butylamino, *N,N-*Dimethylamino, *N,N-*Diethylamino, *N-*Ethyl*-N-*methylamino, *N*-Methyl-*N*-n-propylamino, *N*-iso-Propyl-*N*-n-propylamino und *N*-tert-Butyl-*N-*methylamino. C₁-C₄-Alkylamino steht beispielsweise für einen Monoalkylaminorest mit 1 bis 4 Koh-lenstoffatomen oder für einen Dialkylaminorest mit jeweils 1 bis 4 Kohlenstoffatomen pro Alkylsubstituent.
Alkylcarbonyl steht beispielhaft und vorzugsweise für Methylcarbonyl, Ethylcarbonyl, n-Propylcarbonyl, iso-Propylcarbonyl, n-Butylcarbonyl und tert-Butylcarbonyl.
Alkoxycarbonyl steht beispielhaft und vorzugsweise für Methoxycarbonyl, Ethoxycarbonyl, n-Propoxycarbonyl, iso-Propoxycarbonyl, n-Butoxycarbonyl und tert-Butoxycarbonyl.
Alkylaminocarbonyl steht für einen Alkylaminocarbonylrest mit einem oder zwei (unabhängig voneinander gewählten) Alkylsubstituenten, beispielhaft und vorzugsweise für Methylaminocarbonyl, Ethylaminocarbonyl, n-Propylaminocarbonyl, iso-Propylaminocarbonyl, tert-Butylaminocarbonyl, *N,N-*Dimethylaminocarbonyl, *N,N-*Diethylaminocarbonyl, *N*-Ethyl-*N*-methylaminocarbonyl, *N-*Methyl-*N*-n-propylaminocarbonyl, *N*-iso-Propyl-*N*-n-propylaminocarbonyl und *N*-tert-Butyl-*N-*methylaminocarbonyl. C₁-C₄-Alkylaminocarbonyl steht beispielsweise für einen Monoalkylaminocarbonylrest mit 1 bis 4 Kohlenstoffatomen oder für einen Dialkylaminocarbonylrest mit jeweils 1 bis 4 Kohlenstoffatomen pro Alkylsubstituent.
Alkylcarbonylamino steht beispielhaft und vorzugsweise für Methylcarbonylamino, Ethylcarbonylamino, n-Propylcarbonylamino, iso-Propylcarbonylamino, n-Butylcarbonylamino und tert-Butylcarbonylamino.
Alkylsulfonyl steht beispielhaft und vorzugsweise für Methylsulfonyl, Ethylsulfonyl, n-Propylsulfonyl, iso-Propylsulfonyl, n-Butylsulfonyl und tert-Butylsulfonyl.
Alkylaminosulfonyl steht für einen Alkylaminosulfonylrest mit einem oder zwei (unabhängig voneinander gewählten) Alkylsubstituenten, beispielhaft und vorzugsweise für Methylaminosulfonyl, Ethylaminosulfonyl, n-Propylaminosulfonyl, iso-Propylaminosulfonyl, tert-Butylaminosulfonyl, *NN-*Dimethylaminosulfonyl, *N,N-*Diethylaminosulfonyl, *N*-Ethyl-*N*-methylaminosulfonyl, *N*-Methyl-*N*-n-propylaminosulfonyl, *N-*iso-Propyl-*N-*n-propylaminosulfonyl und *N*-tert-Butyl-*N*-methyl-aminosulfonyl. C₁-C₄-Alkylaminosulfonyl steht beispielsweise für einen Monoalkylaminosulfonylrest mit 1 bis 4 Kohlenstoffatomen oder für einen Dialkylamino-sulfonylrest mit jeweils 1 bis 4 Kohlenstoffatomen pro Alkylsubstituent.
Alkylsulfonylamino steht beispielhaft und vorzugsweise für Methylsulfonylamino, Ethyl-sulfonylamino, n-Propylsulfonylamino, iso-Propylsulfonylamino, n-Butylsulfonylamino und tert-Butylsulfonylamino.
Cycloalkyl steht für eine monocyclische Cycloalkylgruppe mit in der Regel 3 bis 6 Kohlenstoffatomen, beispielhaft und vorzugsweise für Cycloalkyl seien genannt Cyclopropyl, Cyclobutyl, Cyclopentyl und Cyclohexyl.
Heterocyclyl steht für einen monocyclischen, heterocyclischen Rest mit 5 oder 6 Ringatomen und bis zu 3, vorzugsweise bis zu 2 Heteroatomen und/oder Heterogruppen aus der Reihe N, O, S, SO, SO₂, wobei ein Stickstoffatom auch ein N-Oxid bilden kann. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, beispielhaft und vorzugsweise für Pyrrolidin-2-yl, Pyrrolidin-3-yl, Pyrrolinyl, Tetrahydrofuranyl, Tetrahydrothienyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Piperazin-1-yl, Piperazin-2-yl.
Halogen steht für Fluor, Chlor, Brom und Jod, vorzugsweise für Fluor und Chlor.

In den Formeln der Gruppe, die für R¹ stehen kann, steht der Endpunkt der Linie, neben der jeweils ein * steht, nicht für ein Kohlenstoffatom beziehungsweise eine CH₂-Gruppe sondern ist Bestandteil der Bindung zu dem Atom, an das R¹ gebunden ist.

In den Formeln der Gruppe, die für R⁶ stehen kann, steht der Endpunkt der Linie, neben der jeweils ein # steht, nicht für ein Kohlenstoffatom beziehungsweise eine CH₂-Gruppe sondern ist Bestandteil der Bindung zu dem Atom, an das R⁶ gebunden ist.

Bevorzugt sind Verbindungen der Formel (I), in welcher
R¹ für eine Gruppe der Formel 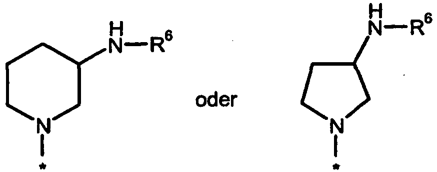 steht,
   wobei
   * die Anknüpfstelle an den Heterocyclus bedeutet,
   R⁶ für Pyrid-2-yl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,2-Pyrazol-5-yl steht,
   wobei Pyrid-2-yl, Pyrimid-2-yl, 1,3-Thiazol-2-yl und 1,3-Thiazol-4-yl substituiert sind mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
   worin Alkyl, Alkylamino, Alkylcarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
   und
   wobei 2-Aminopyrimid-4-yl, 2-Cyclopropylaminopyrimid-4-yl, 2-Methylamino-pyrimid-4-yl, 2-Ethylaminopyrimid-4-yl und 1,2-Pyrazol-5-yl substituiert sein können mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
R² für Phenyl steht,
   wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy, Methyl und Methoxy,
R³ für Wasserstoff steht,
   entweder
R⁴ für Wasserstoff steht,
   und
R⁵ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
   wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   und
   wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
   worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   oder
R⁴ für Trifluormethyl steht,
   und
R⁵ für Cyano steht,
   und ihre Salze, ihre Solvate und die Solvate ihrer Salze.

Bevorzugt sind auch Verbindungen der Formel (I), in welcher
R¹ für eine Gruppe der Formel 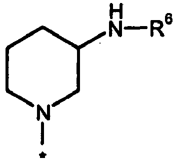 steht,
   wobei
   * die Anknüpfstelle an den Heterocyclus bedeutet,
   R⁶ für Pyrid-2-yl oder 1,3-Thiazol-2-yl steht,
   wobei Pyrid-2-yl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R² für Phenyl steht,
   wobei Phenyl substituiert ist mit 1 bis 2 Substituenten, wobei die Substituenten unabhängig voneinander ausgewählt werden aus der Gruppe bestehend aus Chlor, Fluor, Trifluormethyl, Trifluormethoxy, Methyl und Methoxy,
R³ für Wasserstoff steht,
   entweder
R⁴ für Wasserstoff steht,
   und
R⁵ für Cyano, Trifluormethyl, Hydroxycarbonyl, Methoxycarbonyl, Ethoxycarbonyl, C₁-C₂-Alkylaminocarbonyl, Piperazinylcarbonyl, Piperidinylcarbonyl oder Mopholinylcarbonyl steht,
   wobei Piperazinylcarbonyl, Piperidinylcarbonyl und Mopholinylcarbonyl substituiert sein können mit 1 bis 3 Substituenten, wobei die Substituenten unabhängig voneinander ausgewählt werden aus der Gruppe bestehend aus Oxo, Halogen und C₁-C₄-Alkyl,
   und
   wobei Alkylaminocarbonyl substituiert sein kann mit einem Substituenten, wobei der Substituent ausgewählt wird aus der Gruppe bestehend aus Piperazinyl, Piperidinyl und Mopholinyl,
   worin Piperazinyl, Piperidinyl und Mopholinyl substituiert sein können mit 1 bis 2 Substituenten, wobei die Substituenten unabhängig voneinander ausgewählt werden aus der Gruppe bestehend aus Oxo, Halogen und C₁-C₄-Alkyl,
   oder
R⁴ für Trifluormethyl steht,
   und
R⁵ für Cyano steht,
   und ihre Salze, ihre Solvate und die Solvate ihrer Salze.

Besonders bevorzugt sind Verbindungen der Formel (I), in welcher
R¹ für eine Gruppe der Formel 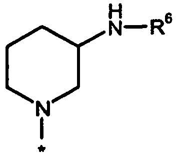 steht,
   wobei
   * die Anknüpfstelle an den Heterocyclus bedeutet,
   R⁶ für eine Gruppe der Formel 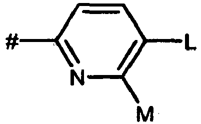 steht,
   wobei
   # die Anknüpfstelle an NH bedeutet,
   L für Cyano, Nitro oder Trifluormethylcarbonyl steht,
   M für Wasserstoff oder Amino steht,
R² für Phenyl steht, wobei Phenyl substituiert ist mit 1 bis 2 Substituenten, wobei die Substituenten unabhängig voneinander ausgewählt werden aus der Gruppe bestehend aus Chlor, Fluor und Methoxy,
R³ für Wasserstoff steht,
   entweder
R⁴ für Wasserstoff steht,
   und
R⁵ für Cyano, Hydroxycarbonyl, Methoxycarbonyl, Methylaminocarbonyl, Dimethylaminocarbonyl, Piperazinylcarbonyl oder Piperidinylcarbonyl steht,
   wobei Piperazinylcarbonyl und Piperidinylcarbonyl substituiert sind mit 1 bis 2 Substituenten, wobei die Substituenten unabhängig voneinander ausgewählt werden aus der Gruppe bestehend aus Halogen und C₁-C₄-Alkyl,
   und
   wobei Methylaminocarbonyl und Dimethylaminocarbonyl substituiert sind mit einem Substituenten Piperidinyl,
   worin Piperidinyl substituiert ist mit 1 bis 2 Substituenten, wobei die Substituenten unabhängig voneinander ausgewählt werden aus der Gruppe bestehend aus Halogen und C₁-C₄-Alkyl,
   oder
R⁴ für Trifluormethyl steht,
   und
R⁵ für Cyano steht,
   und ihre Salze, ihre Solvate und die Solvate ihrer Salze.

Besonders bevorzugt sind Verbindungen der Formel (I), in welcher
R¹ für eine Gruppe der Formel 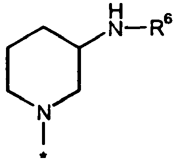 steht,
   wobei
   * die Anknüpfstelle an den Heterocyclus bedeutet,
   R⁶ für eine Gruppe der Formel 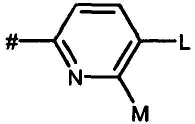 steht,
   wobei
   # die Anknüpfstelle an NH bedeutet,
   entweder
   L für Cyano steht,
   und
   M für Wasserstoff steht,
   oder
   L für Cyano, Nitro oder Trifluormethylcarbonyl steht,
   und
   M für Amino steht,
R² für Phenyl steht,
   wobei Phenyl substituiert ist mit 1 bis 2 Substituenten, wobei die Substituenten unabhängig voneinander ausgewählt werden aus der Gruppe bestehend aus Chlor, Fluor und Methoxy,
R³ für Wasserstoff steht,
   entweder
R⁴ für Wasserstoff steht,
   und
R⁵ für Cyano, Hydroxycarbonyl, Methoxycarbonyl, Methylaminocarbonyl, Dimethylaminocarbonyl, Piperazinylcarbonyl oder Piperidinylcarbonyl steht,
   wobei Piperazinylcarbonyl und Piperidinylcarbonyl substituiert sind mit 1 bis 2 Substituenten, wobei die Substituenten unabhängig voneinander ausgewählt werden aus der Gruppe bestehend aus Halogen und C₁-C₄-Alkyl,
   und
   wobei Methylaminocarbonyl und Dimethylaminocarbonyl substituiert sind mit einem Substituenten Piperidinyl,
   worin Piperidinyl substituiert ist mit 1 bis 2 Substituenten, wobei die Substituenten unabhängig voneinander ausgewählt werden aus der Gruppe bestehend aus Halogen und C₁-C₄-Alkyl,
   oder
R⁴ für Trifluormethyl steht,
   und
R⁵ für Cyano steht,
   und ihre Salze, ihre Solvate und die Solvate ihrer Salze.

Bevorzugt sind auch Verbindungen der Formel (I), in welcher R¹ für eine Gruppe der Formel 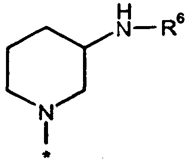 steht, wobei * die Anknüpfstelle an den Heterocyclus bedeutet.

Bevorzugt sind auch Verbindungen der Formel (I), in welcher
R⁶ für Pyrid-2-yl oder 1,3-Thiazol-2-yl steht,
   wobei Pyrid-2-yl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl.

Bevorzugt sind auch Verbindungen der Formel (I), in welcher
R⁶ für eine Gruppe der Formel 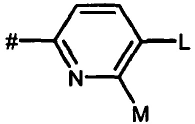 steht,
   wobei
   # die Anknüpfstelle an NH bedeutet,
   L für Cyano, Nitro oder Trifluormethyl steht,
   M für Wasserstoff oder Amino steht.

Bevorzugt sind auch Verbindungen der Formel (I), in welcher R⁶ für 5-Cyanopyrid-2-yl steht.

Bevorzugt sind auch Verbindungen der Formel (I), in welcher R⁶ für 5-Trifluormethylcarbonyl-6-amino-pyrid-2-yl steht.

Bevorzugt sind auch Verbindungen der Formel (I), in welcher
R² für Phenyl steht, wobei Phenyl substituiert ist mit 1 bis 2 Substituenten, wobei die Substituenten unabhängig voneinander ausgewählt werden aus der Gruppe bestehend aus Chlor, Fluor und Methoxy.

Bevorzugt sind auch Verbindungen der Formel (I), in welcher R³ für Wasserstoff steht.

Bevorzugt sind auch Verbindungen der Formel (I), in welcher
entweder
R⁴ für Wasserstoff steht,
   und
R⁵ für Cyano, Hydroxycarbonyl, Methoxycarbonyl, Methylaminocarbonyl, Dimethylaminocarbonyl, Piperazinylcarbonyl oder Piperidinylcarbonyl steht,
   wobei Piperazinylcarbonyl und Piperidinylcarbonyl substituiert sind mit 1 bis 2 Substituenten, wobei die Substituenten unabhängig voneinander ausgewählt werden aus der Gruppe bestehend aus Halogen und C₁-C₄-Alkyl,
   und
   wobei Methylaminocarbonyl und Dimethylaminocarbonyl substituiert sind mit einem Substituenten Piperidinyl,
   worin Piperidinyl substituiert ist mit 1 bis 2 Substituenten, wobei die Substituenten unabhängig voneinander ausgewählt werden aus der Gruppe bestehend aus Halogen und C₁-C₄-Alkyl,
   oder
R⁴ für Trifluormethyl steht,
   und
R⁵ für Cyano steht.

Gegenstand der Erfindung ist weiterhin ein Verfahren zur Herstellung der Verbindungen der Formel (I), oder ihrer Salze, ihrer Solvate oder der Solvate ihrer Salze, wobei entweder
[A] die Verbindungen der Formel 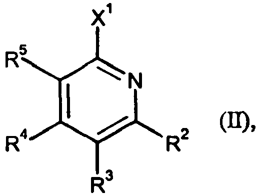 in welcher
   R², R³, R⁴ und R⁵ die oben angegebene Bedeutung haben,
   und
   X¹ für Halogen, bevorzugt Chlor oder Fluor, steht,
   mit Verbindungen der Formel

   R¹-H (III),

   in welcher
   R¹ die oben angegebene Bedeutung hat,
   umgesetzt werden,
   oder
[B] die Verbindungen der Formel 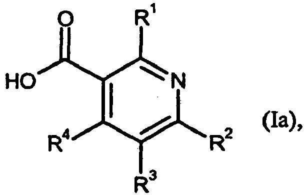 in welcher
   R¹, R², R³ und R⁴ die oben angegebene Bedeutung haben,
   mit Verbindungen der Formel

   R^{5a}-H (IV),

   in welcher
   R^{5a} für C₁-C₄-Alkoxy, C₁-C₄-Alkylamino oder 5- oder 6-gliedriges Heterocyclyl steht, wobei Heterocyclyl substituiert sein kann mit 1 bis 3 Substituenten, wobei die Substituenten unabhängig voneinander ausgewählt werden aus der Gruppe bestehend aus - Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   und
   wobei Alkylamino substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
   worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
   zu Verbindungen der Formel 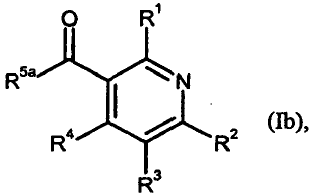 umgesetzt werden.

Die Verbindungen der Formeln (Ia) und (Ib) sind eine Teilmenge der Verbindungen der Formel (I).

Die Umsetzung nach Verfahren [A] erfolgt im Allgemeinen in inerten Lösungsmitteln, gegebenenfalls in Gegenwart einer Base, gegebenenfalls in einer Mikrowelle, bevorzugt in einem Temperaturbereich von 50°C bis 200°C bei Normaldruck bis 5 bar.

Basen sind beispielsweise beispielsweise Alkalicarbonate, wie z.B. Natrium-, Kalium- oder Caesiumcarbonat, oder organische Basen wie Trialkylamine, z.B. Triethylamin, *N*-Methylmorpholin, *N*-Methylpiperidin, 4-Dimethylaminopyridin oder Diisopropylethylamin, oder andere Basen wie beispielsweise Natriumhydrid oder Kalium-tert.-butylat, bevorzugt ist Diisopropylethylamin oder Natriumhydrid.

Inerte Lösungsmittel sind beispielsweise Halogenkohlenwasserstoffe wie Methylenchlorid oder Trichlormethan, Alkohole wie Methanol, Ethanol, n-Propanol oder Isopropanol, oder Ether wie Dioxan oder Tetrahydrofuran, oder andere Lösungsmittel wie beispielsweise Dimethylsulfoxid, Dimethylformamid oder N-Methylpyrrolidon, oder Gemische dieser Lösungsmittel, bevorzugt ist N-Methylpyrrolidon oder Dimethylsulfoxid.

Die Verbindungen der Formel (II) sind bekannt, lassen sich nach bekannten Verfahren aus den entsprechenden Ausgangsverbindungen synthetisieren oder können analog den im Beispielteil (Beispiel 16A und 17A) beschriebenen Verfahren oder analog zu W. Yang et al., Organic Letters 2003, 5, 17, 3131 hergestellt werden.

Die Verbindungen der Formel (III) sind bekannt, lassen sich nach bekannten Verfahren aus den entsprechenden Ausgangsverbindungen synthetisieren oder können analog den im Beispielteil (Beispiel 1A bis 15A) beschriebenen Verfahren hergestellt werden.

Die Umsetzung nach Verfahren [B] erfolgt im Allgemeinen in inerten Lösungsmitteln, in Gegenwart eines Dehydratisierungsreagenzes, gegebenenfalls in Gegenwart einer Base, bevorzugt in einem Temperaturbereich von Raumtemperatur bis zum Rückfluss der Lösungsmittel bei Normaldruck.

Inerte Lösungsmittel sind beispielsweise Halogenkohlenwasserstoffe wie Methylenchlorid, Trichlormethan oder 1,2-Dichlorethan, Ether wie Dioxan, Tetrahydrofuran oder 1,2-Dimethoxyethan, oder andere Lösemittel wie Aceton, Dimethylformamid, Dimethylacetamid, 2-Butanon oder Acetonitril. Ebenso ist es möglich, Gemische der Lösemittel einzusetzen. Bevorzugt ist Dimethylformamid.

Als Dehydratisierungsreagenzien eignen sich hierbei beispielsweise Carbodiimide wie z.B. *N*,*N*'-Diethyl-, *N,N'*-Dipropyl-, *N,N'*-Diisopropyl-, *N,N'*-Dicyclohexylcarbodiimid, *N*-(3-Dimethylamino-isopropyl)-*N*'-ethylcarbodiimid-hydrochlorid (EDC), *N*-Cyclohexylcarbodiimid-*N'-*propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)-phosphoniumhexafluorophosphat, oder *O*-(Benzotriazol-1-yl)-*N,N,N'N'*-tetra-methyluronium-hexafluorophosphat (HBTU), 2-(2-Oxo-1-(2H)-pyridyl)-1,1,3,3-tetramethyluroniumtetrafluoro-borat (TPTU) oder *O*-(7-Azabenzotriazol-1-yl)-*N,N,N',N'*-tetramethyl-uroniumhexafluorophosphat (HATU), oder 1-Hydroxybenzotriazol (HOBt), oder Benzotriazol-1-yloxytris(dimethylamino)-phosphoniumhexafluorophosphat (BOP), oder *N*-Hydroxysuccinimid, oder Mischungen aus diesen, mit Basen.

Basen sind beispielsweise Alkalicarbonate, wie z.B. Natrium- oder Kaliumcarbonat, oder -hydrogencarbonat, oder organische Basen wie Trialkylamine z.B. Triethylamin, *N*-Methylmorpholin, *N*-Methylpiperidin, 4-Dimethylaminopyridin oder Diisopropylethylamin, bevorzugt ist Diisopropylethylamin.

Vorzugsweise wird die Kondensation mit HATU in Gegenwart von Diisopropylethylamin durchgeführt.

Die Verbindungen der Formel (Ia) lassen sich nach Verfahren [A] herstellen. Die Carbonsäure ist während der Reaktion als Methyl- oder Ethylester geschützt, der in der letzten Stufe zur Herstellung der Verbindungen der Formel (Ia) mit einer Base, wie z. B. Natrium-, Kalium- oder Lithiumhydroxid verseift wird.

Die Herstellung der Ausgangsverbindungen und der Verbindungen der Formel (I) kann durch die folgenden Syntheseschemata verdeutlicht werden.

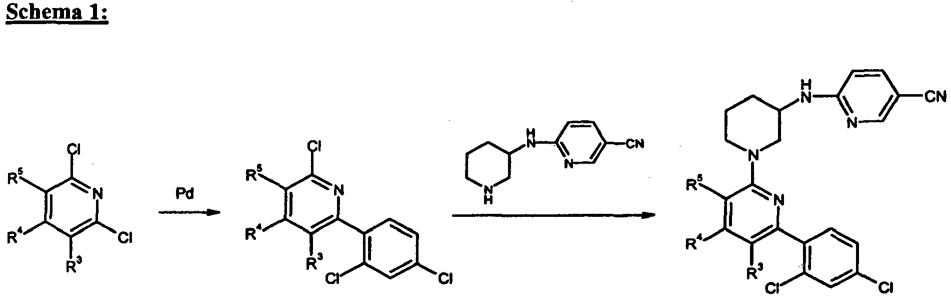

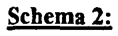

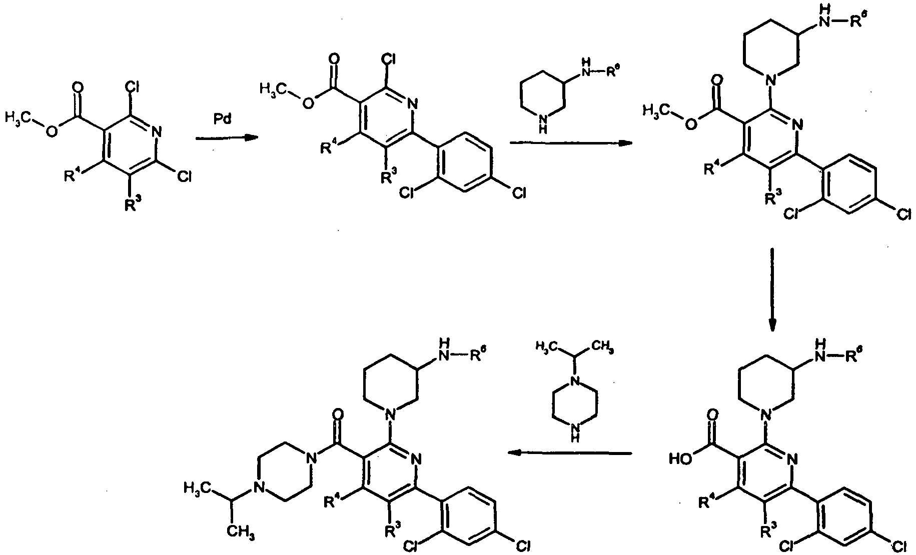

Die erfindungsgemäßen Verbindungen zeigen ein nicht vorhersehbares, wertvolles pharmakologisches und pharmakokinetisches Wirkspektrum.

Sie eigenen sich daher zur Verwendung als Arzneimittel zur Behandlung und/oder Prophylaxe von Krankheiten bei Menschen und Tieren.

Weiterer Gegenstand der vorliegenden Offenbarung ist der Einsatz der erfindungsgemäßen Verbindungen zur Behandlung und/oder Prophylaxe von Erkrankungen, vorzugsweise hämatologischen Erkrankungen, insbesondere von Leukopenien und Neutropenie.

Die erfindungsgemäßen Verbindungen eignen sich daher zur Prophylaxe und/oder Behandlung von neurodegenerativen Erkrankungen wie z.B. Alzheimer, Parkinson, Schizophrenie, Degeneration, Dementia, Depressionen; Aggression, zerebrovaskulär Ischämie, Schlafstörungen, Huntington-Chorea, neurotraumatische Erkrankungen wie z.B. Schlaganfall; Typ 2 Diabetes Mellitus und assoziierte Erkrankungen wie z.B. das metabolische Syndrom oder Fettleibigkeit, Typ 1 Diabetes Mellitus, Diabetische Nephrophatie, Diabetische Neurophatie, Diabetische Retinophatie, Glomerulonephritis, Hyperkalzämie, Hyperglykämie, Hyperlipidimie, Glukose-Galaktose-Malabsorption, allgemeine endokrine Dysfunktionen wie z.B. Pankreatitis; hämatologische Erkrankungen, wie zum Beispiel erworbene und angeborene Neutropenie, medikamentös induzierte Neutropenie, parasitär induzierte Neutropenie, Chemotherapie-induzierte Neutropenie, Granulozytopenie, erworbene und angeborene Leukopenie, erworbene und angeborene Anämie, hämolytische Anämie, Sichelzellenanämie, erworbene und angeborenen Thrombozytopenie, Leukozytenfunktionssörungen, Störungen der Blutgerinnung, Graft-versus-host-Reaktion; Krebs, wie zum Beispiel Mammakarzinom, Kolontumor, gastrointestinale Tumore, Hodgkin-Lymphom, Non-Hodgkin-Lymphom, Kaposisarkom, Lebertumor, Pankreastumor, Hauttumor, Knochenmarkstumor, Leukämien wie z.B. akute lymphatische Leukämie, akute myeloische Leukämie, chronische myeloische Leukämie, chronische lymphatische Leukämie, MLL-Leukämie, Prostatatumore, Lungenkrebs, Nierentumore; Asthma, progrediente, nicht vollständig reversible Obstruktion der Atemwege, Lungenentzündung, Lungenfehlfunktion; Entzündungserkrankungen wie z.B. Autoimmunerkrankungen wie Multiple Sklerose, rheumatoide Arthritis; Infektionen durch gram-negative und gram-positive Bakterien, virale Infektionen, Pilzinfektionen wie z. B. durch Candida albicans, HIV- und HIV-assoziierte Infektionen, Hepatitis der Typen A, B und C, parasitäre Infektionen; Malaria; Haarausfall; verminderte Beweglichkeit von Spermien; Wundheilung; Glaukom; Osteoporose, Knochenmarkserkrankungen, Knochen- und Gelenkserkrankungen; Herzkreislauferkrankungen wie z.B. Herzfehler, Herzinsuffizienz, Herzfibrose, Herzrhythmusstörungen, Myokardinfarkt, Medikamenten- oder Substanzinduzierte Kardiotoxizität, Atherosklerose, Bluthochdruck; Sepsis; inflammatorische Erkrankungen; Pemphigus Vulgaris.

Die erfindungsgemäßen Verbindungen eignen sich besonders zur Prophylaxe und/oder Behandlung von neurodegenerativen Erkrankungen wie z.B. Alzheimer und Schizophrenie, von Typ 2 Diabetes Mellitus und assoziierten Erkrankungen, von Krebs, von Leukopenien und/oder von Neutropenien.

Die erfindungsgemäßen Verbindungen eignen sich besonders zur Prophylaxe und/oder Behandlung von Leukopenien und/oder von Neutropenien.

Die erfindungsgemäßen Verbindungen können darüber hinaus auch zur effizienten *ex vivo* Vermehrung von adulten hämatopoetischen Stammzellen aus dem Knochenmark und/oder aus peripherem Blut und/oder zur *ex vivo* Vermehrung von embryonalen Stammstellen aus Nabelschnurblut eingesetzt werden.

Die erfindungsgemäßen Verbindungen können darüber hinaus auch zur *ex vivo* Vermehrung embryonaler und/oder adulter Stammzellen sowie zur *ex vivo* Differenzierung embryonaler und/oder adulter Stammzellen verwendet werden.

Diese so expandierten Zellen können dann zur Verkürzung der durch myeloablative Therapien induzierten Zytopenien oder im Rahmen von therapeutischen Transplantationsverfahren oder bei hämatologischen Systemerkrankungen, wie z.B. Leukämien, oder mit nach der Expansion gentechnisch veränderter Zellen für Gentherapien verwendet werden.

Weiterer Gegenstand der vorliegenden Offenbarung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Offenbarung ist ein Verfahren zur Behandlung und/oder Prophylaxe von Erkrankungen, insbesondere der zuvor genannten Erkrankungen, unter Verwendung einer therapeutisch wirksamen Menge einer erfindungsgemäßen Verbindung.

Weiterer Gegenstand der vorliegenden Erfindung sind Arzneimittel, enthaltend eine erfindungsgemäße Verbindung und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
Eine Kombination der erfindungsgemäßen Verbindungen mit in der Klinik verwendeten chemotherapeutischen Agenzien können bei unterschiedlichen Tumorerkrankungen zu einem signifikant verbesserten Behandlungserfolg führen. Bei den chemotherapeutischen Agenzien handelt es sich um Substanzen, welche entweder die Teilungsrate von Tumorzellen inhibieren und/oder die Neovaskularisierung von soliden Tumoren unterbindet. Dazu gehören u.a. Substanzen aus der Gruppe der Taxane wie z.B. Paclitaxel, oder Docetaxel, Substanzen, welche die Mitose von Tumorzellen inhibieren wie z.B. Vinblastin, Vincristin, Vindesin oder Vinorelbin. Substanzen aus der Klasse der Platinum-Derivate wie z.B. Cisplatin, Carboplatin, Oxaliplatin, Nedaplatin oder Lobaplatin. Weiterhin gehören zu den chemotherapeutischen Agentien Substanzen aus der Klasse der alkylierenden Agentien, wie z.B. Cyclophosphamid, Ifosfamid, Melphalan, Chlorambucil, Pipobroman, Triethylen-Melamin, Busulfan, Carmustin, Lomustin, Streptozin, Dacarbazin oder Temozolomid. Zu den chemotherapeutischen Agenzien werden auch Anti-Metabolite wie z.B. Folsäure-Antagonisten, Pyrimidin-Analoga, Purin-Analoga oder Adenosin-Desaminase-Inhibitoren gezählt. In diese Substanzklasse gehören u.a. Methotrexat, 5-Fluorouracil, Floxuridin, Cytarabin, Pentostatin und Gemcitabin. Als chemotherapeutische Agenzien werden auch Naturstoffe oder deren Derivate eingesetzt, zu denen u.a. Enzyme, Anti-Tumor-Antikörper und Lymphokine gezählt werden. Dazu gehören z.B. Bleomycin, Dactinomycin, Daunorubicin, Doxorubicin, Epirubicin, Idarubicin, ara-V, Paclitaxel, Mithramycin, Mitomycin-C, L-Asparaginase, Interferone (z.B. IFNalhpa) und Etoposid. Andere chemotherapeutische Agentien mit anti-proliferativer und/oder antiangiogenetischer Wirkung sind Sorafenib, Sunitinib, Bortezomib, DAST Inhibitor (BAY 73-4506), ZK-Epothilon u.a.

Weiterer Gegenstand der vorliegenden Erfindung ist ein Verfahren zur *ex vivo* Vermehrung von adulten hämatopoetischen Stammzellen aus dem Knochenmark und/oder aus peripherem Blut das dadurch gekennzeichnet ist, dass eine wirksame Menge der erfindungsgemäßen Verbindung zugegeben wird.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nicht überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

### Bevorzugt ist die orale Applikation.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (wie beispielsweise Pflaster), Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich als vorteilhaft erwiesen, bei parenteraler Applikation Mengen von etwa 5 bis 1500 mg je 24 Stunden zur Erzielung wirksamer Ergebnisse zu verabreichen. Bei oraler Applikation beträgt die Menge etwa 5 bis 2000 mg je 24 Stunden.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen. Die Angabe "w/v" bedeutet "weight/volume" (Gewicht/Volumen). So bedeutet beispielsweise "10% w/v": 100 ml Lösung oder Suspension enthalten 10 g Substanz.

### A) Beispiele

### Abkürzungen:

- abs.: absolut
- Boc: *tert*-Butoxycarbonyl
- CDCl₃: Deuterochloroform
- d: Tag
- DIEA: *N,N*-Diisopropylethylamin
- DMAP: 4-*N,N-*Dimethylaminopyridin
- DMF: Dimethylformamid
- DMSO: Dimethylsulfoxid
- d. Th.: der Theorie
- EDC: *N'*-(3-Dimethylaminopropyl)-*N-*ethylcarbodiimid × HCl
- eq.: Äquivalent
- ESI: Elektrospray-Ionisation (bei MS)
- ges.: gesättigt
- h: Stunde
- HOBt: 1-Hydroxy-1H-benzotriazol × H₂O
- HPLC: Hochdruck-, Hochleistungsflüssigchromatographie
- konz.: konzentriert
- LC-MS: Flüssigchromatographie-gekoppelte Massenspektrometrie
- min.: Minuten
- MS: Massenspektrometrie
- MW: Molekulargewicht [g/mol]
- NMR: Kernresonanzspektroskopie
- OAc: Acetat
- OEt: Ethoxy
- p.a.: zur Analyse
- PyBOP: 1-Benzotriazolyloxy-tripyrrolidinophosphoniumhexafluorophosphat
- R_{f}: Retentionsindex (bei DC)
- RP-HPLC: Reverse Phase HPLC
- RT: Raumtemperatur
- Rₜ: Retentionszeit (bei HPLC)
- TBTU: (Benzotriazol-1-yloxy)bisdimethylaminomethyliumfluoroborat
- TFA: Trifluoressigsäure
- THF: Tetrahydrofuran

### LC-MS Methoden:

Methode 1: Instrument: Micromass Quattro LCZ mit HPLC Agilent Serie 1100; Säule: Phenomenex Synergi 2.5 µ MAX-RP 100A Mercury 20 mm x 4 mm; Eluent A: 1 1 Wasser + 0.5 ml 50%ige Ameisensäure, Eluent B: 1 1 Acetonitril + 0.5 ml 50%ige Ameisensäure; Gradient: 0.0 min 90%A → 0.1 min 90%A → 3.0 min 5%A → 4.0 min 5%A → 4.1 min 90%A; Fluss: 2 ml/min; Ofen: 50°C; UV-Detektion: 208- 400 nm.

Methode 2: Gerätetyp MS: Micromass ZQ; Gerätetyp HPLC: Waters Alliance 2795; Säule: Merck Chromolith SpeedROD RP-18e 100 mm x 4.6 mm; Eluent A: Wasser + 500 µl 50%ige Ameisensäure / l; Eluent B: Acetonitril + 500 µl 50%ige Ameisensäure / l; Gradient: 0.0 min 10%B→ 7.0 min 95%B→ 9.0 min 95%B; Ofen: 35 °C; Fluss: 0.0 min 1.0 ml/min→ 7.0 min 2.0 ml/min→ 9.0 min 2.0 ml/min; UV-Detektion: 210 nm

Methode 3: Gerätetyp MS: Micromass ZQ; Gerätetyp HPLC: HP 1100 Series; UV DAD; Säule: Phenomenex Gemini 3µ 30 mm x 3.00 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.

Methode 4: Instrument: Micromass Platform LCZ mit HPLC Agilent Serie 1100; Säule: Thermo Hypersil GOLD 3µ 20 mm x 4 mm; Eluent A: 1 l Wasser + 0.5 ml 50%ige Ameisensäure , Eluent B: 1l Acetonitril + 0.5 ml 50%ige Ameisensäure; Gradient: 0.0 min 100%A → 0.2 min 100%A → 2.9 min 30%A → 3.1 min 10%A →5.5 min 10%A; Ofen: 50°C; Fluss: 0.8 ml/min; UV-Detektion: 210 nm.

Methode 5: Gerätetyp MS: Waters ZQ; Gerätetyp HPLC: Waters Alliance 2795; Säule: Phenomenex Onyx Monolithic C18, 100 mm x 3 mm; Eluent A: 1 1 Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 210 nm.

Methode 6: Gerätetyp MS: Micromass ZQ; Gerätetyp HPLC: Waters Alliance 2795; Säule: Phenomenex Synergi 2.5µ MAX-RP 100A Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 0.1 min 90%A → 3.0 min 5%A → 4.0 min 5%A → 4.01 min 90%A; Fluss: 2 ml/min;; Ofen: 50°C; UV-Detektion: 210 nm.

Methode 7: Instrument: Micromass Quattro LCZ mit HPLC Agilent Serie 1100; Säule: Phenomenex Onyx Monolithic C18, 100 mm x 3 mm. Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2 min 65%A → 4.5 min 5%A → 6 min 5%A; Fluss: 2 ml/min; Ofen: 40°C; UV-Detektion: 208-400 nm.

Methode 8: Instrument: Micromass QuattroPremier mit Waters UPLC Acquity; Säule: Thermo Hypersil GOLD 1.9µ 50 mm × 1mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.1 min 100%A → 1.5 min 10%A → 2.2 min 10%A; Ofen: 50°C; Fluss: 0.33 ml/min; UV-Detektion: 210 nm.

Methode 9: Instrument: Micromass Quattro Micro MS mit HPLC Agilent Serie 1100; Säule: Thermo Hypersil GOLD 3µ 20 mm x 4 mm; Eluent A: 1 l Wasser + 0.5 ml 50%ige Ameisensäure, Eluent B: 1 1 Acetonitril + 0.5 ml 50%ige Ameisensäure; Gradient: 0.0 min 100%A → 3.0 min 10% A → 4.0 min 10%A → 4.01 min 100%A → 5.00 min 100%A; Fluss: 0.0 min/3.0 min/4.0 min/4.01 min 2.5 ml/min, 5.00 min 2 ml/min; Ofen: 50°C; UV-Detektion: 210 nm.

Methode 10: Präparative HPLC: Säule: Reprosil C18; Gradient: Acetonitril/Wasser mit 0.1% Salzsäure.

Methode 11: Präparative HPLC: Säule: Reprosil C18; Gradient: Acetonitril/Wasser mit 0.1% Trifluoressigsäure.

Methode 12: Instrument: Waters ACQUITY SQD UPLC System; Säule: Waters Acquity UPLC HSS T3 1.8µ, 50 mm x 1 mm; Eluent A: 1 l Wasser + 0.25 ml 99%ige Ameisensäure, Eluent B: 1 l Acetonitril + 0.25 ml 99%ige Ameisensäure; Gradient: 0.0 min 90%A → 1.2 min 5%A → 2.0 min 5%A; Fluss: 0.40 ml/min; Ofen: 50°C; UV-Detektion: 210 - 400 nm.

Methode 13: Präparative HPLC: Säule: Reprosil C18; Gradient: Acetonitril/Wasser.

Als Mikrowellenreaktor wurde ein "single mode" Gerät vom Typ Emrys™ Optimizer verwendet.

### Ausgangsverbindungen

### Beispiel 1A

### tert-Butyl-(6-chlorpyridin-2-yl)carbamat

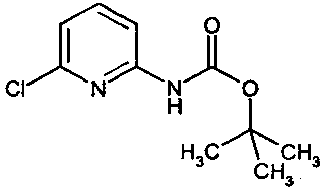

23.4 g (181.8 mmol) 2-Chlor-5-aminopyridin wurden unter Argon mit 150 ml THF versetzt und auf 0°C gekühlt. Es wurden 73.3 g (400 mmol) Bis-(trimethylsilyl)-natriumamid und 43.65 g (200 mmol) Di-tert-butyldicarbonat, gelöst in 150 ml THF, zugetropft. Nach 15 min wurde das Kühlbad entfernt und weitere 15 min bei RT nachgerührt. Das THF wurde abrotiert und der Rückstand mit Essigsäureethylester und 0.5 N Salzsäure versetzt und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Dichlormethan/Methanol 100% → 100:3). Es wurden 36.54 g (88% d. Th.) des Produktes als Feststoff erhalten.

LCMS (Methode 3): Rₜ = 2.41 min. (m/z = 175 (M+H)⁺).

¹H-NMR (400MHz, DMSO-d₆): δ = 10.11 (s, 1H), 7.78 (d, 2H), 7.1 (t, 1H), 1.47 (s, 9H).

### Beispiel 2A

### tert-Butyl-(6-chlor-3-formylpyridin-2-yl)carbamat

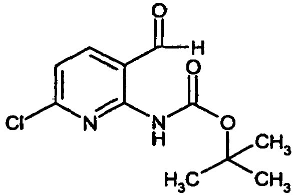

Die Reaktionsapparatur wurde ausgeheizt, und die Reaktion erfolgte unter Argon und wurde gerührt. 15 g (65.6 mmol) tert-Butyl-(6-chlorpyridin-2-yl)carbamat (Beispiel 1A) und 19 g (164 mmol) 1,2-Bis(dimethylamino)ethan wurden in 270 ml THF vorgelegt und auf -78°C abgekühlt. Es wurden 102.5 ml (164 mmol) Butyllithium (1.6N) zugetropft. Nach Beendigung des Zutropfens wurde die Reaktion langsam auf -10°C erwärmt und bei -10°C für 2 h gehalten. Dann wieder auf -78°C abgekühlt, und es wurden 10 ml (131 mmol) DMF dazugegeben. Die Reaktion wurde langsam auf RT erwärmt und das Reaktionsgemisch wurde auf 1 l Essigsäureethylester und 350 ml 1N Salzsäure gegeben, 15 min nachgerührt und die organische Phase wurde abgetrennt. Es wurde mit Wasser und gesättigter Natriumhydrogencarbonat-Lösung gewaschen, über Magnesiumsulfat getrocknet und am Rotationsverdampfer eingeengt. Der Rückstand wurde mit Diethylether versetzt und der Feststoff wurde abgesaugt und nachgetrocknet. Es wurden 12.3 g (73% d. Th.) des Produktes als Feststoff erhalten.

LCMS (Methode 3): Rₜ = 2.19 min. (m/z = 255 (M+H)⁻).

¹H-NMR (400MHz, DMSO-d₆): δ = 10.37 (s, 1H), 9.83 (s, 1H), 8.2 (d, 1H), 7.42 (d, 1H), 1.46 (s, 9H).

### Beispiel 3A

### tert-Butyl-{6-chlor-3-[(hydroxyimino)methyl]pyridin-2-yl}carbamat

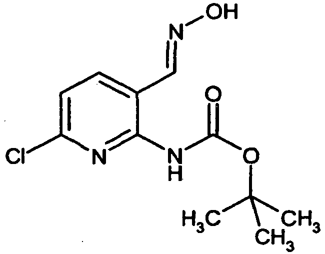

15.45 g (60.2 mmol) tert-Butyl-(6-chlor-3-formylpyridin-2-yl)carbamat (Beispiel 2A) wurde in 750 ml Ethanol vorgelegt und mit einer Lösung aus 225 ml Wasser und 9.38 g (120.4 mmol) Natriumacetat versetzt und 5 min gerührt. Eine Lösung aus 225 ml Wasser und 8.36 g (114.4 mmol) Hydroxylamin Hydrochlorid wurde zugegeben und 4 h bei RT gerührt. Bei 20°C wurde das Reaktionsgemisch am Rotationsverdampfer eingeengt. Der Rückstand wurde in Essigsäureethylester aufgenommen, zweimal mit gesättigter Natriumhydrogencarbonat-Lösung und einmal mit gesättiger Natriumchlorid-Lösung gewaschen. Die organische Phase wurde abgetrennt, über Magnesiumsulfat getrocknet und bei 20°C am Rotationsverdampfer eingeengt. Es wurden 15.5 g (80% d. Th.) des Produktes als Feststoff erhalten.

LCMS (Methode 3): Rₜ = 2.08 min. (m/z = 270 (M+H)⁻).

¹H-NMR (400MHz, DMSO-d₆): δ = 11.71 (s, 1H), 9.91 (s, 1H), 8.14 (s, 1H), 8.02 (d, 1H), 7.3 (d, 1H), 1.49 (s, 9H).

### Beispiel 4A

### 2-Amino-6-chlorpyridin-3-carbaldehydoxim Hydrochlorid

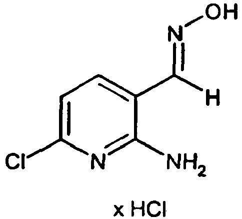

15.5 g (57 mmol) tert-Butyl-{6-chlor-3-[(hydroxyimino)methyl]pyridin-2-yl}carbamat (Beispiel 3A) wurden in 285 ml 4N Chlorwasserstoff in Dioxan gelöst und 30 min nachgerührt. Man engte das Reaktionsgemisch um die Hälfte ein und gab den gleichen Teil an Diethylether zu. Das Reaktionsgemisch wurde 20 min nachgerührt und das Produkt abfiltriert und mit Diethylether nachgewaschen. Es wurden 11g (94% d. Th.) des Produktes als Feststoff erhalten.

LCMS (Methode 6): Rₜ = 1.09 min. (m/z = 172 (M+H)⁺)

¹H-NMR (400MHz, DMSO-d₆): δ = 8.27 (s, 1H), 7.61 (d, 1H), 6.65 (d, 1H).

### Beispiel 5A

### 2-Amino-6-chlorpyridin-3-carbonitril

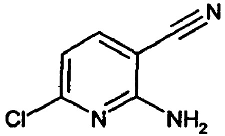

11.15 g (53.6 mmol) 2-Amino-6-chlorpyridin-3-carbaldehydoxim Hydrochlorid (Beispiel 4A) wurden in Dioxan vorgelegt, mit 13 ml (161 mmol) Pyridin versetzt und auf 0°C abgekühlt. Es wurden 8.3 ml (58.95 mmol) Trifluoressigsäureanhydrid zugegeben, man erwärmte die Reaktion auf RT und rührte anschließend 2 h bei 60°C. Das Reaktionsgemisch wurde in einem Gemisch von Essigsäureethylester und Natriumhydrogencarbonat-Lösung aufgenommen. Die organische Phase wurde mit gesättigter wässriger Natriumchlorid-Lösung gewaschen, über Magnesiumsulfat getrocknet und am Rotationsverdampfer eingeengt. Der Rückstand wurde in Dichlormethan:Diethylether 3:1 suspendiert, und der Feststoff wurde abgesaugt und getrocknet. Es wurden 5.56 g (66% d. Th.) des Produktes als Feststoff erhalten.

LCMS (Methode 6): Rₜ = 1.0 min. (m/z = 154 (M+H)⁺).

¹H-NMR (400MHz, DMSO-d₆): δ = 7.91 (d, 1H), 7.38 (s, 2H), 6.69 (d, 1H).

### Beispiel 6A

### 4-(Trifluoracetyl)morpholin

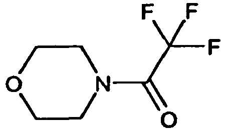

15 g (172 mmol) Morpholin wurden in 750 ml Dichlormethan vorgelegt, und es wurden bei 0°C 29 ml (206 mmol) Trifluoressigsäureanhydrid und 119 ml (688 mmol) N,N-Diisopropylethylamin zugegeben. Das Reaktionsgemisch wurde auf RT erwärmt und 3 h bei RT nachgerührt. Das Reaktionsgemisch wurde eingeengt und der Rückstand in Essigsäureethylester aufgenommen und nacheinander mit wässriger Natriumhydrogencarbonat-Lösung, 1N Salzsäure und wieder mit wässriger Natriumhydrogencarbonat-Lösung gewaschen. Die organische Phase wurde über Magnesiumsulfat getrocknet und am Rotationsverdampfer eingeengt. Es wurden 28 g (88% d. Th.) des Produktes als Öl erhalten.

LCMS (Methode 9): Rₜ = 1.22 min. (m/z = 184 (M+H)⁺)

¹H-NMR (400MHz, DMSO-d₆): δ = 3.65 (m, 2H), 3.56 (m, 2H).

### Beispiel 7A

### tert-Butyl-[6-chlor-3-(trifluoracetyl)pyridin-2-yl]carbamat

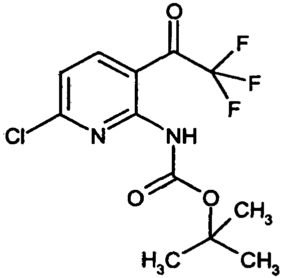

8 g (35 mmol) tert-Butyl-(6-chlorpyridin-2-yl)carbamat (Beispiel 1A) wurden in 100 ml THF vorgelegt und auf -50°C gekühlt. Es wurden 55 ml (87 mmol) Butyllithium (1.6N) zugetropft. Nach Beendigung des Zutropfens wurde die Reaktion langsam auf -10°C erwärmt und bei 0°C für 2 h gerührt. Anschließend wurde wieder auf -40°C abgekühlt, und es wurden 12.8 g (70 mmol) 4-(Trifluoracetyl)morpholin (Beispiel 6A), gelöst in 4 ml THF, zugegeben. Die Reaktionslösung wurde 1 h bei -40°C nachgerührt, danach bei -40°C auf 1 l Essigsäureethylester und 350 ml Ammoniumchloridlösung gegossen und extrahiert. Die organische Phase wurde abgetrennt, über Magnesiumsulfat getrocknet und am Rotationsverdampfer eingeengt. Man chromatographierte das Reaktionsgemisch an Kieselgel (Laufmittel Cyclohexan/ Essigsäureethylester 10:1). Es wurden 9 g (79% d. Th.) des Produktes als Öl erhalten.

¹H-NMR (400MHz, DMSO-d₆): δ = 10.96 (s, 1H), 7.99 (d, 1H), 7.4 (d, 1H), 1.43 (s, 9H).

### Beispiel 8A

### tert-Butyl-3-[(5-cyanopyridin-2-yl)amino]piperidin-1-carboxylat

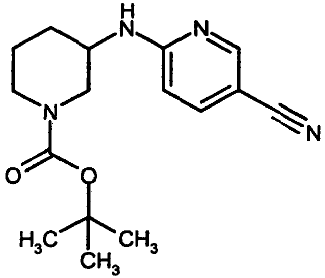

1.0 g (4.99 mmol) tert-Butyl-3-aminopiperidin-1-carboxylat und 1.383 g (9.99 mmol) 6-Chlorpyridin-3-carbonitril und 1.29 g (9.99 mmol) Diisopropylethylamin wurden in 40 ml DMSO suspendiert und für 45 min in einem Mikrowellenreaktor auf 140°C erhitzt. Der Ansatz wurde durch Kugelrohrdestillation weitgehend vom DMSO befreit, mit Wasser versetzt und der ausfallende Niederschlag abfiltriert. Nach Trocknen im Hochvakuum erhielt man 2.24 g (46% d. Th.) des Produktes.

LCMS (Methode 3): Rₜ = 2.23 min. (m/z = 303 (M+H)⁺).

### Beispiel 9A

### tert-Butyl-3-[(6-amino-5-cyanopyridin-2-yl)amino]piperidin-1-carboxylat

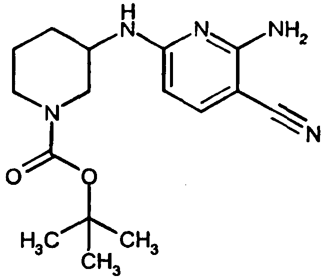

2.15 g (10.7 mmol) tert-Butyl-3-aminopiperidin-1-carboxylat, 1.50 g (9.77 mmol) 2-Amino-6-chlorpyridin-3-carbonitril (Beispiel 5A) und 1.89 g (14.7 mmol) Diisopropylethylamin wurden in 6 ml DMSO suspendiert und für 8 h in einem Mikrowellenreaktor auf 130°C erhitzt. Das Reaktionsgemisch wurde mit Essigsäureethylester (100 ml) und Wasser (40 ml) verdünnt, die organische Phase wurde getrennt und mit gesättigter wässriger Natriumchlorid-Lösung (50 ml) gewaschen, über Magnesiumsulfat getrocknet und eingeengt. Der Rückstand wurde chromatographiert auf Kieselgel (Laufmittel: Cyclohexan-Essigsäureethylester 4:1 bis 1:1). Es wurden 2.04 g (60% d. Th.) des Produktes als Feststoff isoliert.

LCMS (Methode 6): Rₜ = 1.69 min. (m/z = 318 (M+H)⁺)

### Beispiel 10A

### 6-(Piperidin-3-ylamino)pyridin-3-carbonitril Hydrochlorid

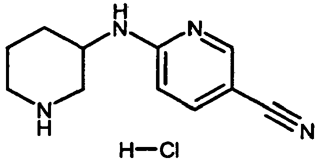

In 4.3 ml einer Salzsäurelösung in Dioxan (4 M) wurden 2.24 g (7.4 mmol) tert-Butyl-3-[(5-cyanopyridin-2-yl)amino]piperidin-1-carboxylat (Beispiel 8A) gelöst und 3 h bei RT gerührt. Nach vollständiger Reaktion wurde das Lösungsmittel komplett entfernt. Es wurden 1.74 g (90% d. Th.) des Produktes als Feststoff erhalten.

LCMS (Methode 8): Rₜ = 0.27 min. (m/z = 203 (M+H)⁺)

¹H-NMR (400MHz, DMSO-d₆): δ = 9.13 (m, 1H), 9.0 (m, 1H), 8.44 (d, 1H), 7.89 (m, 1H), 7.74 (dd, 1H), 6.63 (d, 1H), 5.58 (s, br), 4.19 (s, br, 1H), 3.57 (s, 1H), 3.34 (d, 1H), 3.14 (d, 1H), 2.88 (m, 1H), 2.7-2.81 (m, 1H), 1.82-2.0 (m, 2H), 1.63-1.79 (m, 1H), 1.48-1.59 (m, 1H).

### Beispiel 11A

### 2-Amino-6-(piperidin-3-ylamino)pyridin-3-carbonitril Hydrochlorid

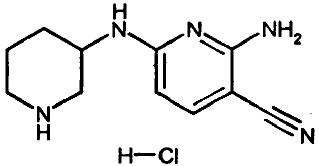

In 40 ml einer Salzsäurelösung in Dioxan (4 M) wurden 2.00 g (6.3 mmol) tert-Butyl-3-[(6-amino-5-cyanopyridin-2-yl)amino]piperidin-1-carboxylat (Beispiel 9A) gelöst und 2 h bei RT gerührt. Nach vollständiger Reaktion wurde das Lösungsmittel zur Hälfte eingeengt, und 20 ml Diethylether wurden zugegeben. Der Niederschlag wurde abfiltriert und getrocknet. Es wurden 1.80 g (100% d. Th.) des Produktes als Feststoff erhalten.

LCMS (Methode 8): Rₜ = 0.25 min. (m/z = 218 (M+H)⁺)

¹H-NMR (400MHz, DMSO-d₆): δ = 9.38 (br m, 1H), 8.97 (br m, 1H), 8.25 (br m, 1H), 7.53 (m, 1H), 7.40 (br s, 2H), 6.01 (d, 1H), 4.16 (br m, 1H), 3.34 (br m, 1H), 3.10 (m, 1H), 2.89 (m, 2H), 2.00-1.84 (m, 2H), 1.73 (m, 1H), 1.55 (m, 1H).

### Beispiel 12A

### tert-Butyl-3-({6-[(tert-butoxycarbonyl)amino]-5-(trifluoracetyl)pyridin-2-yl}amino)piperidin-1-carboxylat

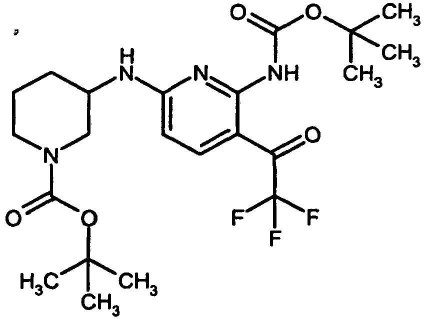

561 mg (2.8 mmol) tert-Butyl-3-aminopiperidin-1-carboxylat, 700 mg (2.16 mmol) tert-Butyl-[6-chlor-3-(trifluoracetyl)pyridin-2-yl]carbamat (Beispiel 7A) und 0.56 ml (3.23 mmol) Diisopropylethylamin wurden in 14 ml DMSO suspendiert und für 45 min in einem Mikrowellenreaktor auf 90°C erhitzt. Das Reaktionsgemisch wurde mit Essigsäureethylester (100 ml) verdünnt und mit gesättigter wässriger Ammoniumchloridlösung (3 x 40 ml) und dann mit gesättigter wässriger Natriumhydrogencarbonatlösung (40 ml) gewaschen. Die organische Phase wurde getrocknet über Magnesiumsulfat und eingeengt. Der Rückstand wurde chromatographiert auf Kieselgel (Laufmittel: Cyclohexan-Essigsäureethylester 5:1 bis 1:1). Es wurden 670 mg (63% d. Th.) des Produktes isoliert.

LCMS (Methode 6): Rₜ = 2.70 min. (m/z = 489 (M+H)⁺)

### Beispiel 13A

### 1-[2-Amino-6-(piperidin-3-ylamino)pyridin-3-yl]-2,2,2-trifluorethanon Hydrochlorid

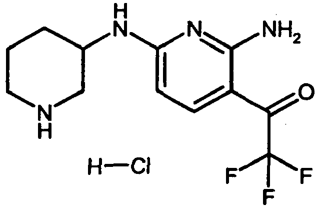

In 25 ml einer Salzsäurelösung in Dioxan (4 M) wurden 670 mg (1.37 mmol) tert-Butyl-3-({6-[(tert-butoxycarbonyl)amino]-5-(trifluoracetyl)pyridin-2-yl}amino)piperidin-1-carboxylat (Beispiel 12A) gelöst und 20 h bei RT gerührt. Nach vollständiger Reaktion wurde das Reaktionsmischung mit Diethylether (100 ml) verdünnt, und der Niederschlag wurde abfiltriert und mit Diethylether (100 ml) gewaschen und getrocknet. Es wurden 286 mg (64% d. Th.) des Produktes als Feststoff erhalten.

LCMS (Methode 6): Rₜ = 0.81 min. (m/z = 289 (M+H)⁺)

¹H-NMR (400MHz, DMSO-d₆): δ = 9.26 (br s, 1H), 9.07 (br s, 1H), 8.8.34 (br s, 1H), 7.59 (d, 1H), 6.22 (br, 2H), 6.03 (d, 1H), 4.25 (br m, 1H), 3.36 (m, 1H), 3.13 (m, 1H), 2.93 (m, 2H), 2.00-1.85 (m, 2H), 1.73 (m, 1H), 1.56 (m, 1H).

### Beispiel 14A

### tert-Butyl-3-[(6-amino-5-nitropyridin-2-yl)amino]piperidin-1-carboxylat

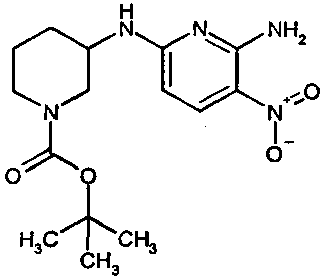

500 mg (2.11 mmol) tert-Butyl-3-aminopiperidin-1-carboxylat, 772 mg (4.22 mmol) 2-Amino-6-chlor-3-nitropyridin und 1.05 ml (6.34 mmol) Diisopropylethylamin wurden in 18 ml DMSO suspendiert und für 45 min in einem Mikrowellenreaktor auf 120°C erhitzt. Das Reaktionsgemisch wurde mittels präparativer reverse-phase HPLC aufgereinigt (Methode 13). Es wurden 600 mg (81 % d. Th.) des Produktes als Feststoff isoliert.

LCMS (Methode 6): Rₜ = 1.77 min. (m/z = 338 (M+H)⁺)

### Beispiel 15A

### 3-Nitro-N⁶-(piperidin-3-yl)pyridin-2,6-diamin Hydrochlorid

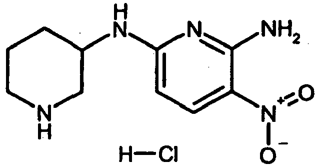

In 40 ml einer Salzsäurelösung in Dioxan (4 M) wurden 610 mg (1.62 mmol) tert-Butyl-3-[(6-amino-5-nitropyridin-2-yl)amino]piperidin-1-carboxylat (Beispiel 14A) gelöst und 30 min bei RT gerührt. Nach vollständiger Reaktion wurde das Lösungsmittel komplett entfernt. Es wurden 662 mg des Rohproduktes erhalten.

LCMS (Methode 4): Rₜ = 0.86 min. (m/z = 238 (M+H)⁺)

### Beispiel 16A

### Methyl-2-chlor-6-(2,4-dichlorphenyl)pyridin-3-carboxylat

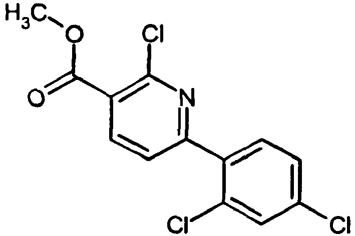

In 120 ml entgastem THF wurden 6 g (29.12 mmol) Methyl-2,6-dichlorpyridin-3-carboxylat, 5.56 g (29.12 mmol) 2,4-Dichlorphenylboronsäure, 12.07 g (87.4 mmol) Kaliumcarbonat und dann 1.68 g (1.46 mmol) Tetrakis(triphenylphosphin)palladium(0) vorgelegt und der Ansatz für 16 h unter Rückflußbedingungen und unter einer Argonathmosphäre erhitzt. Es wurden 300 ml Wasser und 500 ml Essigsäureethylester zugegeben. Nach Trennung der Phasen und Entfernen des Lösungsmittels wurde der Rückstand an Kieselgel chromatographiert (Cyclohexan/Essigsäureethylester 6:1). Es wurden 2.5 g (27% d. Th.) des Produktes als Feststoff isoliert.

LCMS (Methode 6): Rₜ = 2.41 min. (m/z = 316 (M+H)⁺)

¹H-NMR (400MHz, DMSO-d₆): δ = 8.38 (d, 1H), 7.86 (d, 1H), 7.82 (d, 1H), 7.68 (d, 1H), 7.60 (dd, 1H), 3.92 (s, 3H).

### Beispiel 17A

### Methyl-2-chlor-6-(2-methoxyphenyl)pyridin-3-carboxylat

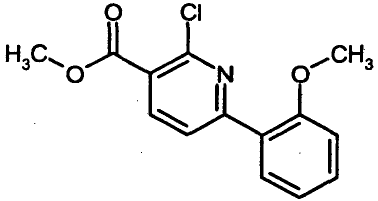

In einem Gemisch aus 20 ml Methanol und 8 ml Toluol wurden 500 mg (1.90 mmol) 2-Chlor-6-(2-methoxyphenyl)pyridin-3-carbonsäure vorgelegt und mit 8 ml (16 mmol) einer 2 molaren Lösung von Trimethylsilyldiazomethan in Hexan versetzt. Man rührte bei RT für 15 h und gab dann 1 ml Essigsäure zu. Das Lösungsmittel wurde vollständig im Vakuum entfernt und der erhaltene Feststoff ohne weitere Aufreinigung eingesetzt. Es wurden 502 mg (87% d. Th.) des Produktes erhalten.

LCMS (Methode 8): Rₜ = 1.27 min. (m/z = 278 (M+H)⁺)

¹H-NMR (400MHz, DMSO-d₆): δ = 8.29 (d, 1H), 8.04 (d, 1H), 7.81 (dd, 1H), 7.50 (dt, 1H), 7.21 (d, 1H), 7.11 (t, 1H), 3.90 (s, 3H), 3.88 (s, 3H).

### Ausführungsbeispiele

### Beispiel 1

### 2-{3-[(6-Amino-5-nitropyridin-2-yl)amino]piperidin-1-yl}-6-(4-chorphenyl)pyridin-3-carbonitril

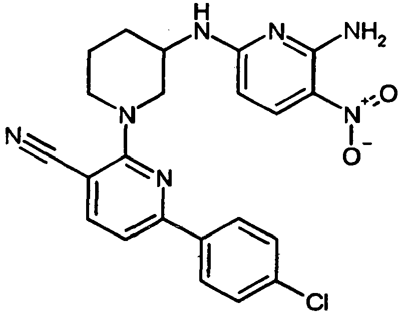

70.4 mg (0.27 mmol) 2-Chlor-6-(4-chlorphenyl)pyridin-3-carbonitril, 100 mg (0.32 mmol) 3-Nitro-N⁶-(piperidin-3-yl)pyridin-2,6-diamin Hydrochlorid (Beispiel 15A) und 0.187 ml (1.08 mmol) N,N-Diisopropylethylamin wurden in 3 ml DMSO vorgelegt. Der Ansatz wurde bei 140°C für 30 min in einer Mikrowelle erhitzt. Das Rohprodukt wurde mittels präparativer HPLC (Methode 13) gereinigt. Man erhielt 99 mg (74% d. Th.) des Produktes als Feststoff.

LCMS (Methode 8): Rₜ = 1.45 min. (m/z = 450 (M+H)⁺)

¹H-NMR (400MHz, DMSO-d₆): δ = 8.13 (d, 2H), 8.09 (d, 2H), 7.89-7.98 (m, 2H), 7.66 (br s, 1H), 7.51 (ψt, 3H), 5.95 (d, 1H), 4.12-4.28 (m, 2H), 3.97-4.06 (m, 1H), 3.36-3.49 (m, 2H), 1.9-2.1 (m, 2H), 1.55-1.77 (m, 2H).

Die Enantiomerentrennung von 2-{3-[(6-Amino-5-nitropyridin-2-yl)amino]piperidin-1-yl}-6-(4-chlorphenyl)pyridin-3-carbonitril (Beispiel 1) wurde unter den folgenden Bedingungen durchgeführt:
Eine Probe von Beispiel 1 (78 mg) wurde in 8 ml Methanol, 8 ml Acetonitril und 15 ml tert-Butylmethylether gelöst und über eine Daicel Chiralpak IA, 5µm, 250 mm x 20 mm Säule chromatographiert (Fluß: 15 ml/min; Detektion bei 220 nm; Injektionsvolumen: 500 µl; Eluent: tert-Butylmethylether:Methanol (90:10), Temperatur: 30°C). Es wurden zwei Fraktionen isoliert:

### Beispiel Ent-A-1: Es wurden 37 mg Produkt in > 99% ee isoliert.

Retentionszeit 4.31 min, spez. Drehwert: [α]²⁰₅₈₉= -113.0° (c = 0.475 g/100 ml Ethanol)

**Beispiel Ent-B-1:** Es wurden 33 mg Produkt in > 98% ee isoliert.

Retentionszeit 4.66 min

### Beispiel 2

### 2-(3-{[6-Amino-5-(trifluoracetyl)pyridin-2-yl]amino}piperidin-1-yl)-6-(2,4-dichlorphenyl)pyridin-3-carbonitril

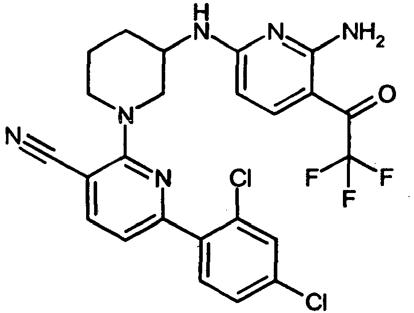

29.5 mg (0.1 mmol) 2-Chlor-6-(2,4-dichlorphenyl)pyridin-3-carbonitril, 30 mg (0.1 mmol) 1-[2-Amino-6-(piperidin-3-ylamino)pyridin-3-yl]-2,2,2-trifluorethanon Hydrochlorid (Beispiel 13A) und 0.09 ml (0.52 mmol) N,N-Diisopropylethylamin wurden in 1 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 35 mg (63% d. Th.) des Produktes als Feststoff.

LCMS (Methode 3): Rₜ = 3.13 min. (m/z = 535 (M+H)⁺)

¹H-NMR (400MHz, DMSO-d₆): δ = 8.50 (br s, 2H), 8.16 (d, 1H), 7.97 (d, 1H), 7.73(d, 1H), 7.62 (d, 1H), 7.45-7.53 (m, 2H), 7.18 (d, 1H), 5.96 (d, 1H), 4.12-4.25 (m, 2H), 3.99-4.09 (m, 1H), 3.2-3.37 (m, 2H), 1.87-2.08 (m, 2H), 1.5-1.74 (m, 2H).

Die Enantiomerentrennung von 2-(3-{[6-Amino-5-(trifluoracetyl)pyridin-2-yl]amino}piperidin-1-yl)-6-(2,4-dichlorphenyl)pyridin-3-carbonitril (Beispiel 2) wurde unter den folgenden Bedingungen durchgeführt:
Eine Probe von Beispiel 2 (190 mg) wurde in 10 ml 2-Propanol gelöst und über eine Daicel Chiralpak AS-H, 5µm, 250 mm x 20 mm Säule chromatographiert (Fluß: 15 ml/min; Detektion bei 220 nm; Injektionsvolumen: 1000 µl; Eluent: iso-Hexan:2-Propanol (50:50), Temperatur: 40°C). Es wurden zwei Fraktionen isoliert:
   **Beispiel Ent-A-2:** Es wurden 73 mg Produkt in > 99% ee isoliert.
   Retentionszeit 6.09 min
   **Beispiel Ent-B-2:** Es wurden 110 mg Produkt in > 99% ee isoliert.
   Retentionszeit 13.38 min

### Beispiel 3

### 2-{3-[(5-Cyanopyridin-2-yl)amino]piperidin-1-yl}-6-(4-fluorphenyl)pyridin-3-carbonitril

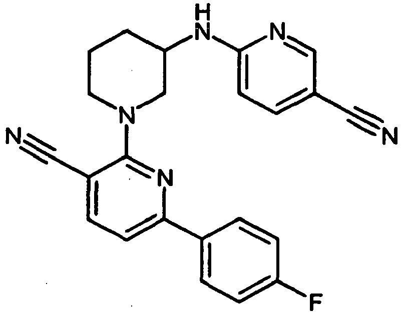

60 mg (0.26 mmol) 2-Chlor-6-(4-fluorphenyl)pyridin-3-carbonitril, 77 mg (0.31 mmol) 6-(Piperidin-3-ylamino)pyridin-3-carbonitril Hydrochlorid (Beispiel 10A) und 0.225 ml (1.29 mmol) N,N-Diisopropylethylamin wurden in 2 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 99 mg (50% d. Th.) des Produktes als Feststoff.

LCMS (Methode 8): Rₜ = 1.40 min. (m/z = 399 (M+H)⁺)

¹H-NMR (400MHz, DMSO-d₆): δ = 8.38 (d, 1H), 8.15 (d, 1H), 8.14 (d, 1H), 8.09 (d, 1H), 7.6-7.68 (m, 2H), 7.46 (d, 1H), 7.32 (ψt, 2H), 6.56 (d, 1H), 4.26 (dd, 1H), 4.01-4.14 (m, 2H), 3.33-3.47 (m, 2H), 2.0-2.08 (m, 1H), 1.88-1.97 (m, 1H), 1.55-1.75 (m, 2H).

### Beispiel 4

### 2-(3-{[6-Amino-5-(trifluoracetyl)pyridin-2-yl]amino}piperidin-1-yl)-6-(4-fluorphenyl)pyridin-3-carbonitril

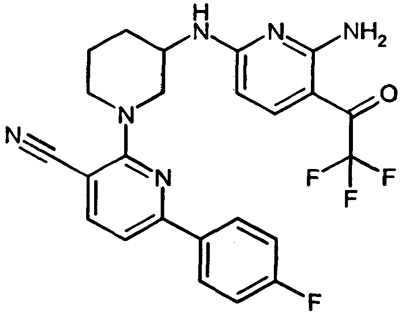

55 mg (0.24 mmol) 2-Chlor-6-(4-fluorphenyl)pyridin-3-carbonitril, 83 mg (0.28 mmol) 1-[2-Amino-6-(piperidin-3-ylamino)pyridin-3-yl]-2,2,2-trifluorethanon Hydrochlorid (Beispiel 13A) und 0.206 ml (1.18 mmol) N,N-Diisopropylethylamin wurden in 2 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 72 mg (54% d. Th.) des Produktes als Feststoff.

LCMS (Methode 8): Rₜ = 1.52 min. (m/z = 485 (M+H)⁺)

¹H-NMR (400MHz, DMSO-d₆): δ = 8.28 (br s, 1H), 7.86-7.94 (m, 3H), 7.74 (d, 1H), 7.37(br s, 1H), 7.22-7.30 (m, 2H), 7.05 (t, 2H), 5.74 (d, 1H), 4.02 (m, 1H), 3.93 (d, 1H), 3.76-3.85 (m, 1H), 3.14-3.25 (m, 2H), 1.78-1.86 (m, 1H), 1.67-1.77 (m, 1H), 1.32-1.55 (m, 2H).

### Beispiel 5

### 2-Amino-6-({1-[3-cyano-6-(4-fluorphenyl)pyridin-2-yl]piperidin-3-yl}amino)pyridin-3-carbonitril

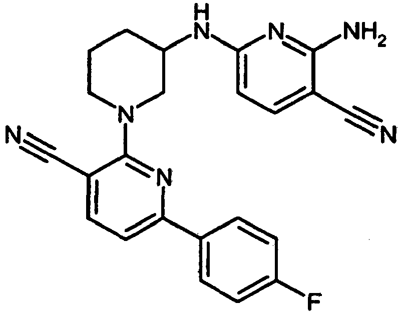

55 mg (0.24 mmol) 2-Chlor-6-(4-fluorphenyl)pyridin-3-carbonitril, 74 mg (0.28 mmol) 2-Amino-6-(piperidin-3-ylamino)pyridin-3-carbonitril Hydrochlorid (Beispiel 11A) und 0.206 ml (1.18 mmol) N,N-Diisopropylethylamin wurden in 2 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 47 mg (48% d. Th.) des Produktes als Feststoff.

LCMS (Methode 3): Rₜ = 2.64 min. (m/z = 414 (M+H)⁺)

¹H-NMR (400MHz, DMSO-d₆): δ = 8.09-8.17 (m, 3H), 7.47 (d, 1H), 7.26-7.36 (m, 3H), 7.14 (br s, 1H), 6.26 (s, 1H), 5.80 (d, 1H), 4.25 (d, 1H), 4.01-4.12 (m, 2H), 3.17-3.28 (m, 2H), 1.97-2.05 (m, 1H), 1.86-1.96 (m, 1H), 1.62-1.75 (m, 1H), 1.49-1.61 (m, 1H).

### Beispiel 6

### 2-Amino-6-({1-[3-cyano-6-(2,4-dichlorphenyl)pyridin-2-yl]piperidin-3-yl}amino)pyridin-3-carbonitril

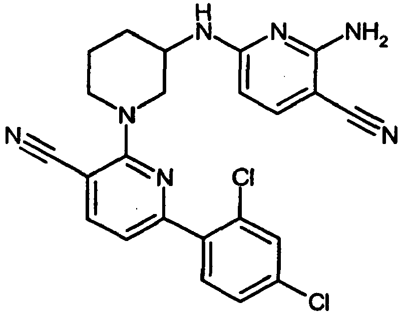

55 mg (0.19 mmol) 2-Chlor-6-(2,4-dichlorphenyl)pyridin-3-carbonitril, 60.9 mg (0.23 mmol) 2-Amino-6-(piperidin-3-ylamino)pyridin-3-carbonitril Hydrochlorid (Beispiel 11A) und 0.169 ml (0.97 mmol) N,N-Diisopropylethylamin wurden in 2 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 30 mg (33% d. Th.) des Produktes als Feststoff.

LCMS (Methode 3): Rₜ = 2.90 min. (m/z = 464 (M+H)⁺)

¹H-NMR (400MHz, DMSO-d₆): δ = 8.14 (d, 1H), 7.75 (s, 1H), 7.63 (d, 1H), 7.54 (dd, 1H), 7.23 (d, 1H), 7.16 (d, 1H), 7.10 (br s, 1H), 6.22 (br s, 2H), 5.79 (d, 1H), 4.28 (d, 1H), 4.09 (m, 1H), 3.91-4.03 (m, 1H), 3.22 (t, 1H), 3.05 (t, 1H), 1.95-2.04 (m, 1H), 1.82-1.92 (m, 1H), 1.6-1.71 (m, 1H), 1.46-1.58 (m, 1H).

Die Enantiomerentrennung von 2-Amino-6-({1-[3-cyano-6-(2,4-dichlorphenyl)pyridin-2-yl]-piperidin-3-yl}amino)pyridin-3-carbonitril (Beispiel 6) wurde unter den folgenden Bedingungen durchgeführt:
Eine Probe von Beispiel 6 (63 mg) wurde in 5 ml Ethanol gelöst und über eine Daicel Chiralpak AS-H, 5µm, 250 mm x 20 mm Säule chromatographiert (Fluß: 15 ml/min; Detektion bei 220 nm; Injektionsvolumen: 2000 µl; Eluent: iso-Hexan:Ethanol (50:50), Temperatur: 40°C). Es wurden zwei Fraktionen isoliert:
   **Beispiel Ent-A-6:** Es wurden 25 mg Produkt in > 99% ee isoliert.
   Retentionszeit 7.33 min
   **Beispiel Ent-B-6:** Es wurden 25 mg Produkt in > 99% ee isoliert.
   Retentionszeit 15.6 min

### Beispiel 7

### 2-{3-[(5-Cyanopyridin-2-yl)amino]piperidin-1-yl}-6-(2,4-dichlorphenyl)pyridin-3-carbonitril

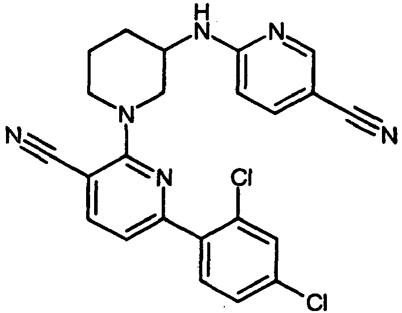

60 mg (0.21 mmol) 2-Chlor-6-(2,4-dichlorphenyl)pyridin-3-carbonitril, 63 mg (0.25 mmol) 6-(Piperidin-3-ylamino)pyridin-3-carbonitril Hydrochlorid (Beispiel 10A) und 0.184 ml (1.06 mmol) N,N-Diisopropylethylamin wurden in 2 ml DMSO vorgelegt. Der Ansatz wurde bei 120°C für 30 min in einer Mikrowelle erhitzt. Das Rohprodukt wurde mittels präparativer HPLC (Methode 13) gereinigt. Man erhielt 61 mg (64% d. Th.) des Produktes als Feststoff.

LCMS (Methode 3): Rₜ = 3.00 min. (m/z = 449 (M+H)⁺)

¹H-NMR (400MHz, DMSO-d₆): δ = 8.32 (d, 1H), 8.12 (d, 1H), 7.74 (d, 1H), 7.52-7.63 (m, 4H), 7.13 (d, 1H), 6.54 (d, 1H), 4.20 (d, 1H), 3.98-4.08 (m, 2H), 3.32-3.42 (m, 2H), 1.98-2.06 (m, 1H), 1.86-1.95 (m, 1H), 1.55-1.73 (m, 2H).

### Beispiel 8

### Methyl-2-(3-{[6-amino-5-(trifluoracetyl)pyridin-2-yl]amino}piperidin-1-yl)-6-(2,4-dichlorphenyl)-pyridin-3-carboxylat Hydrochlorid

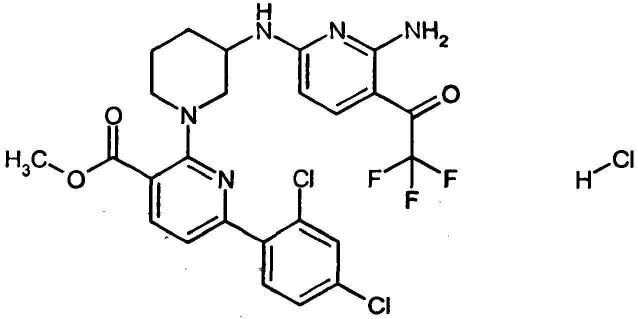

255 mg (0.533 mmol) Methyl-2-chlor-6-(2,4-dichlorphenyl)pyridin-3-carboxylat (Beispiel 16A), 259 mg (0.799 mmol) 1-[2-Amino-6-(piperidin-3-ylamino)pyridin-3-yl]-2,2,2-trifluorethanon Hydrochlorid (Beispiel 13A) und 0.56 ml (3.2 mmol) N,N-Diisopropylethylamin wurden in 4.5 ml DMSO vorgelegt. Der Ansatz wurde bei 140°C für 45 min in einer Mikrowelle erhitzt. Das Rohprodukt wurde mittels präparativer HPLC (Methode 10) gereinigt. Man erhielt 28 mg (8% d. Th.) des Produktes als Feststoff.

LCMS (Methode 6): Rₜ = 2.87 min. (m/z = 568 (M+H)⁺)

¹H-NMR (400MHz, DMSO-d₆): δ = 8.50 (br s, 1H), 8.0 (d, 1H), 7.89 (d, 1H), 7.71(d, 1H), 7.64 (d, 1H), 7.45-7.56 (m, 2H), 7.10 (d, 1H), 5.94 (d, 1H), 4.15 (m, 1H), 3.9-4.0 (m, 1H), 3.84 (s, 3H), 3.58 (m, 1H), 2.96 (dd, 2H), 1.96-2.08 (m, 1H), 1.75-1.86 (m, 1H), 1.57-1.72 (m, 1H), 1.43-1.56 (m, 1H).

### Beispiel 9

### 2-(3-{[6-Amino-5-(trifluoracetyl)pyridin-2-yl]amino}piperidin-1-yl)-6-(2,4-dichlorphenyl)pyridin-3-carbonsäure Hydrochlorid

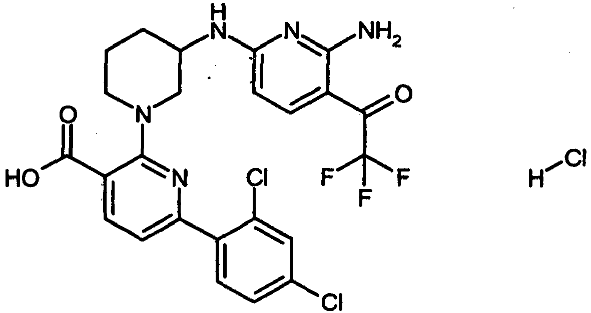

220 mg (0.36 mmol) Methyl-2-(3-{[6-amino-5-(trifluoracetyl)pyridin-2-yl]amino}piperidin-1-yl)-6-(2,4-dichlorphenyl)pyridin-3-carboxylat Hydrochlorid (Beispiel 8) wurden in 10 ml 1,2-Dimethoxyethan gelöst und 4.4 ml Wasser zugegeben. Dann wurden 43 mg (1.1 mmol) Natriumhydroxid zugegeben und 30 min bei RT gerührt. Zur Aufarbeitung wurden 35 ml 2N Salzsäure zugegeben und der sich bildende Niederschlag wurde abgesaugt. Man erhielt 180 mg (75% d. Th.) des Produktes als Feststoff.

LCMS (Methode 6): Rₜ = 2.42 min. (m/z = 554 (M+H)⁺)

¹H-NMR (400MHz, DMSO-d₆): δ =13.1 (br s, 1H), 8.50 (br s, 1H), 8.02 (d, 1H), 7.9-7.96 (m, 1H), 7.71 (d, 1H), 7.62-7.70 (m, 1 H), 7.64 (d, 1 H), 7.47-7.55 (m, 2H), 7.11 (d, 1 H), 5.95 (d, 1 H), 4.11-4.24 (m, 1H), 4.00 (d, 1H), 3.66-3.75 (m, 1H), 2.90 (dd, 2H), 1.98-2.07 (m, 1H), 1.75-1.85 (m, 1H), 1.59-1.74 (m, 1H), 1.42-1.55 (m, 1H).

### Beispiel 10

### Methyl-2-(3-{[6-amino-5-(trifluoracetyl)pyridin-2-yl]amino}piperidin-1-yl)-6-(2-methoxyphenyl)-pyridin-3-carboxylat Hydrochlorid

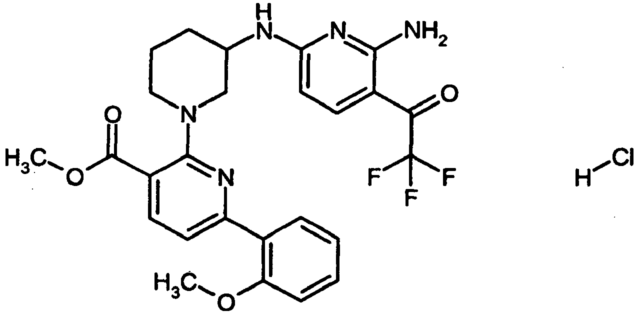

Analog der Herstellung von Beispiel 8 wurden aus 255 mg (0.84 mmol) Methyl-2-chlor-6-(2-methoxyphenyl)pyridin-3-carboxylat (Beispiel 17A) und 299 mg (0.92 mmol) 1-[2-Amino-6-(piperidin-3-ylamino)pyridin-3-yl]-2,2,2-trifluorethanon Hydrochlorid (Beispiel 13A) nach Reinigung des Rohproduktes mittels präparativer HPLC (Methode 10) 275 mg (58% d. Th.) des Produktes als Feststoff erhalten.

LCMS (Methode 6): Rₜ = 2.62 min. (m/z = 530 (M+H)⁺)

¹H-NMR (400MHz, DMSO-d₆): δ = 8.51 (br s, 1H), 7.9-8.0 (m, 2H), 7.77 (d, 1H), 7.55-7.71 (m, 1H), 7.51 (d, 1H), 7.39 (t, 1H), 7.36 (d, 1H), 7.13 (d, 1H), 7.0 (t, 1H), 5.95 (d, 1H), 4.19 (m, 1H), 3.91-3.98 (m, 1H), 3.83 (s, 3H), 3.55-3.65 (m, 1H), 3.49 (s, 3H), 2.96 (t, 2H), 1.98-2.05 (m, 1H), 1.78-1.85 (m, 1H), 1.6-1.71 (m, 1H), 1.44-1.56 (m, 1H).

### Beispiel 11

### 2-(3-{[6-Amino-5-(trifluoracetyl)pyridin-2-yl]amino}piperidin-1-yl)-6-(2-methoxyphenyl)pyridin-3-carbonsäure Hydrochlorid

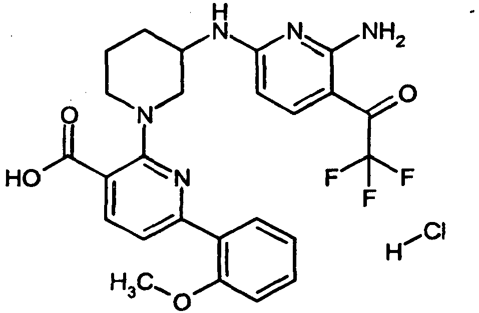

Analog der Herstellung von Beispiel 9 wurden aus 235 mg (0.42 mmol) Methyl-2-(3-{[6-amino-5-(trifluoracetyl)pyridin-2-yl]amino} piperidin-1-yl)-6-(2-methoxyphenyl)pyridin-3-carboxylat Hydrochlorid (Beispiel 10) durch Hydrolyse mit Natriumhydroxid nach Reinigung des Rohproduktes mittels präparativer HPLC (Methode 10) 207 mg (97% d. Th.) des Produktes als Feststoff erhalten.

LCMS (Methode 8): Rₜ = 1.28 min. (m/z = 516 (M+H)⁺)

¹H-NMR (400MHz, DMSO-d₆): δ =13.1 (s, br, 1H), 8.51 (s, br, 1H), 7.98 (d, 2H), 7.92 (d, 1H), 7.78 (dd, 1H), 7.68 (s, br, 1H), 7.51 (m, 1H), 7.35-7.44 (m, 2H), 7.13 (d, 1H), 7.02 (t, 1H), 5.95 (d, 1H), 4.20 (m, 1H), 3.94 (m, 1H), 3.83 (s, 3H), 3.69 (m, 1H), 2.87-2.98 (m, 2H), 1.98-2.07 (m, 1H), 1.76-1.85 (m, 1H), 1.6-1.75 (m, 1H), 1.42-1.54 (m, 1H).

### Allgemeine Versuchsbeschreibung für Amidkupplungen:

0.117 mmol des jeweiligen Amins werden in 3 ml DMF vorgelegt und nacheinander versetzt mit 0.125 mmol HATU, 0.25 mmol N,N-Diisopropylethylamin und 0.083 mmol der Säure (Beispiel 9 bzw. Beispiel 11). Es wird 16 h bei RT nachrührt. Nach Aufreinigung mittels präparativer HPLC (Methode 13) erhält man die Produkte als Feststoff.

Nach der allgemeinen Versuchsbeschreibung für Amidkupplungen wurden die folgenden Verbindungen hergestellt:

| **Bsp.** | **Struktur** | **Charakterisierung** |
|---|---|---|
| **12** | 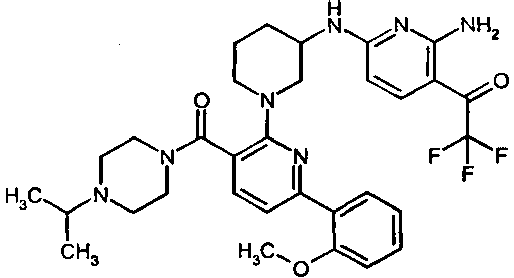 | LC/MS (Methode 8): Rₜ = 1.11 min, (m/z = 624 (M-H)⁺) |
| **13** | 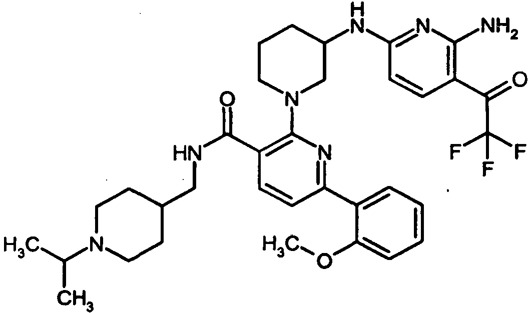 | LC/MS (Methode 3): Rₜ = 1.83 min, (m/z = 654 (M+H)⁺) |
| **14** | 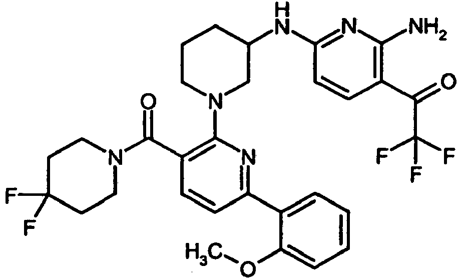 | LC/MS (Methode 8): Rₜ = 1.49 min, (m/z = 619 (M+H)⁺) |
| **15** | 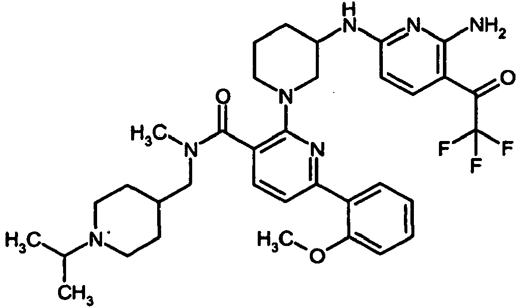 | LC/MS (Methode 6): Rₜ = 1.56 min, (m/z = 668 (M+H)⁺) |
| **16** | 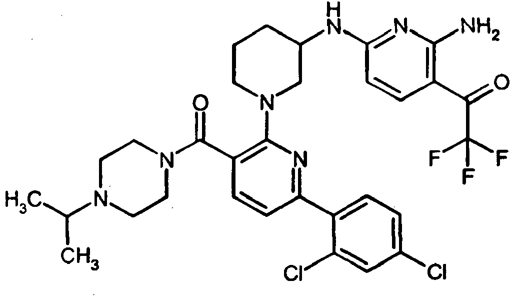 | LC/MS (Methode 6): Rₜ = 1.63 min, (m/z = 664 (M-H)⁺) |
| **17** | 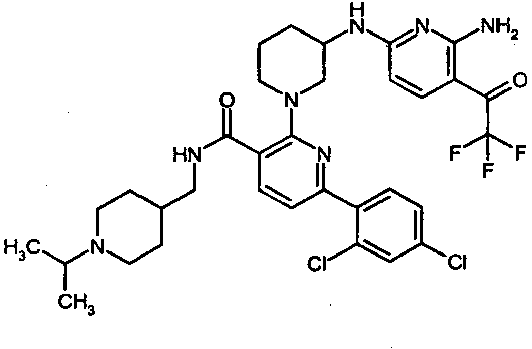 | LC/MS (Methode 8): Rₜ = 1.27 min, (m/z = 691 (M-H)⁺) |
| **18** | 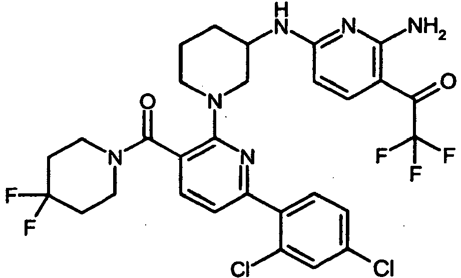 | LC/MS (Methode 6): Rₜ = 2.76 min, (m/z = 657 (M+H)⁺) |
| **19** | 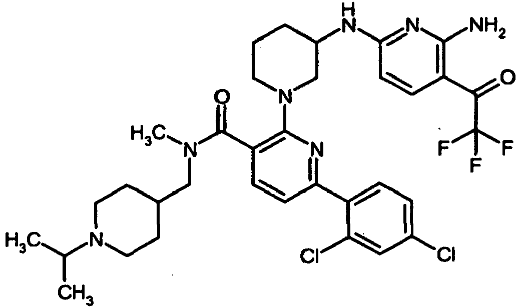 | LC/MS (Methode 8): Rₜ = 1.27 min, (m/z=706 (M+H)⁺) |

Die Enantiomerentrennung von 1-[2-Amino-6-({1-[6-(2-methoxyphenyl)-3-{-[4-(propan-2-yl)-piperazin-1-yl]carbonyl}pyridin-2-yl]piperidin-3-yl}amino)pyridin-3-yl]-2,2,2-trifluorethanon (Beispiel 12) wurde unter den folgenden Bedingungen durchgeführt:
Eine Probe von Beispiel 12 (24 mg) wurde in 2 ml 2-Propanol gelöst und über eine Daicel Chiralpak AD-H, 5µm, 250 mm x 20 mm Säule chromatographiert (Fluß: 15 ml/min; Detektion bei 220 nm; Injektionsvolumen: 500 µl; Eluent: iso-Hexan:2-Propanol mit 0.2% Diethylamin (60:40), Temperatur: 30°C). Es wurden zwei Fraktionen isoliert:
   **Beispiel Ent-A-12:** Es wurden 15 mg Produkt in > 99% ee isoliert.
Retentionszeit 5.26 min, spez. Drehwert: [α]²⁰₅₈₉= -173.7° (c = 0.37 g/100 ml Ethanol)
   **Beispiel Ent-B-12:** Es wurden 9 mg Produkt in > 99% ee isoliert.
   Retentionszeit 6.30 min

Die Enantiomerentrennung von 2-(3-{[6-Amino-5-(trifluoracetyl)pyridin-2-yl]amino}piperidin-1-yl)-6-(2-methoxyphenyl)-N-{[1-(propan-2-yl)piperidin-4-yl]methyl}pyridin-3-carboxamid (Beispiel 13) wurde unter den folgenden Bedingungen durchgeführt:
Eine Probe von Beispiel 13 (28 mg) wurde in 1 ml Ethanol gelöst und über eine Daicel Chiralpak AD-H, 5µm, 250 mm x 20 mm Säule chromatographiert (Fluß: 15 ml/min; Detektion bei 220 nm; Injektionsvolumen: 500 µl; Eluent: iso-Hexan:Ethanol mit 0.2% Diethylamin (60:40), Temperatur: 30°C). Es wurden zwei Fraktionen isoliert:
   **Beispiel Ent-A-13:** Es wurden 6 mg Produkt in > 99% ee isoliert.
Retentionszeit 4.57 min
   **Beispiel Ent-B-13:** Es wurden 12 mg Produkt in > 99% ee isoliert.
Retentionszeit 6.66 min, spez. Drehwert: [α]²⁰₅₈₉= -103.7° (c = 0.375 g/100 ml Ethanol)

Die Enantiomerentrennung von 1-{2-Amino-6-[(1-{3-[(4,4-difluorpiperidin-1-yl)carbonyl]-6-(2-methoxyphenyl)pyridin-2-yl}piperidin-3-yl)amino]pyridin-3-yl}-2,2,2-trifluorethanon (Beispiel 14) wurde unter den folgenden Bedingungen durchgeführt:
Eine Probe von Beispiel 14 (28 mg) wurde in 2 ml 2-Propanol gelöst und über eine Daicel Chiralpak AD-H, 5µm, 250 mm x 20 mm Säule chromatographiert (Fluß: 15 ml/min; Detektion bei 220 nm; Injektionsvolumen: 500 µl; Eluent: iso-Hexan:2-Propanol mit 0.2% Diethylamin (60:40), Temperatur: 30°C). Es wurden zwei Fraktionen isoliert:
   **Beispiel Ent-A-14:** Es wurden 15 mg Produkt in > 99% ee isoliert.
   Retentionszeit 5.96 min, spez. Drehwert: [α]²⁰₅₈₉= -166.7° (c = 0.38 g/100 ml Ethanol)
   **Beispiel Ent-B-14:** Es wurden 11 mg Produkt in > 99% ee isoliert.
   Retentionszeit 7.25 min

### Beispiel 20

### 2-(3-{[6-Amino-5-(trifluoracetyl)pyridin-2-yl]amino}piperidin-1-yl)-6-(2-chlorphenyl)-4-(trifluormethyl)pyridin-3-carbonitril

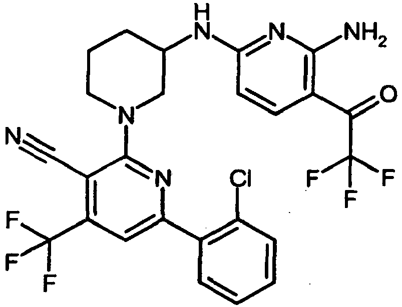

70 mg (0.221 mmol) 2-Chlor-6-(2-chlorphenyl)-4-(trifluormethyl)pyridin-3-carbonitril, 65 mg (0.221 mmol) 1-[2-Amino-6-(piperidin-3-ylamino)pyridin-3-yl]-2,2,2-trifluorethanon Hydrochlorid (Beispiel 13A) und 0.192 ml (1.1 mmol) N,N-Diisopropylethylamin wurden in 2 ml DMSO vorgelegt. Das Reaktionsgemisch wurde für 30 min bei 120°C in einem Mikrowellenreaktor bestrahlt.. Das Reaktionsgemisch wurde mittels präparativer HPLC (Methode 13) gereinigt. Man erhielt 86 mg (68% d. Th.) des Produktes.

LCMS (Methode 6): Rₜ = 2.84 min. (m/z = 569 (M+H)⁺)

¹H-NMR (400MHz, DMSO-d₆): δ = 8.49 (br s, 1H), 7.90 (d, 1H), 7.69 (dd, 1H), 7.59 (d, 1H), 7.42-7.58 (m, 5H), 5.91 (d, 1H), 4.11-4.22 (m, 2H), 3.92-4.01 (m, 1H), 3.48-3.64 (m, 2H), 1.96-2.09 (m, 2H), 1.58-1.75 (m, 2H).

Die Enantiomerentrennung von 2-(3-{[6-Amino-5-(trifluoracetyl)pyridin-2-yl]amino}piperidin-1-yl)-6-(2-chlorphenyl)-4-(trifluormethyl)pyridin-3-carbonitril (Beispiel 20) wurde unter den folgenden Bedingungen durchgeführt:
Eine Probe von Beispiel 20 (75 mg) wurde in 2 ml Ethanol gelöst und über eine Daicel Chiralpak AS-H, 5µm, 250 mm x 20 mm Säule chromatographiert (Fluß: 15 ml/min; Detektion bei 220 nm; Injektionsvolumen: 1000 µl; Eluent: iso-Hexan:Ethanol (70:30), Temperatur: 40°C). Es wurden zwei Fraktionen isoliert:
   **Beispiel Ent-A-20:** Es wurden 28 mg Produkt in > 99% ee isoliert.
   Retentionszeit 4.23 min, spez. Drehwert: [α]²⁰₅₈₉= +318.4° (c = 0.622 g/100 ml Ethanol)
   **Beispiel Ent-B-20:** Es wurden 46 mg Produkt in > 99% ee isoliert.
   Retentionszeit 6.34 min

### Beispiel 21

### 2-(3-{[6-Amino-5-(trifluoracetyl)pyridin-2-yl]amino}piperidin-1-yl)-6-(4-fluorphenyl)-4-(trifluormethyl)pyridin-3-carbonitril

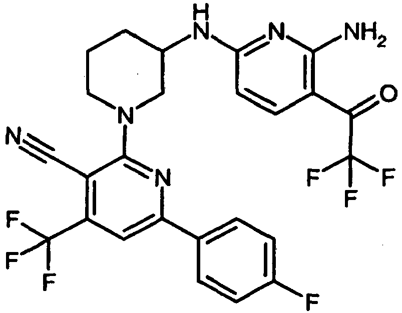

Analog der Herstellung von Beispiel 20 wurden aus 60 mg (0.2 mmol) 2-Chlor-6-(4-fluorphenyl)-4-(trifluormethyl)pyridin-3-carbonitril und 70.4 mg (0.24 mmol) 1-[2-Amino-6-(piperidin-3-ylamino)pyridin-3-yl]-2,2,2-trifluorethanon Hydrochlorid (Beispiel 13A) nach Trennung mittels präparativer HPLC (Methode 13) 92 mg (83% d. Th.) des Produktes als Feststoff erhalten.

LCMS (Methode 8): Rₜ = 1.61 min. (m/z = 553 (M+H)⁺)

¹H-NMR (400MHz, DMSO-d₆): δ = 8.50 (br s, 1H), 8.20-8.28 (m, 2H), 7.90 (d, 1H), 7.78 (s, 1H), 7.58 (br s, 1H), 7.45 (d, 1H), 7.30 (ψt, 2H), 5.90 (d, 1H), 4.17-4.28 (m, 1H), 4.07 (d, 1H), 3.88-3.97 (m, 1H), 3.75-3.83 (m, 1H), 3.59-3.68 (m, 1H), 1.97-2.09 (m, 2H), 1.62-1.77 (m, 2H).

Die Enantiomerentrennung von 2-(3-{[6-Amino-5-(trifluoracetyl)pyridin-2-yl]amino}piperidin-1-yl)-6-(4-fluorphenyl)-4-(trifluormethyl)pyridin-3-carbonitril (Beispiel 21) wurde unter den folgenden Bedingungen durchgeführt:
Eine Probe von Beispiel 21 (84 mg) wurde in 20 ml iso-Hexan:Ethanol (4:1) gelöst und über eine Daicel Chiralpak AS-H, 5µm, 250 mm x 20 mm Säule chromatographiert (Fluß: 20 ml/min; Detektion bei 230 nm; Injektionsvolumen: 20000 µl; Eluent: iso-Hexan:Ethanol (80:20), Temperatur: 24°C). Es wurden zwei Fraktionen isoliert:
   **Beispiel Ent-A-21:** Es wurden 39 mg Produkt in > 99% ee isoliert.
   Retentionszeit 4.52 min, spez. Drehwert: [α]²⁰₅₈₉= +327.6° (c = 0.5905 g/100 ml Ethanol)
   **Beispiel Ent-B-21:** Es wurden 24 mg Produkt in > 99% ee isoliert.
   Retentionszeit 6.62 min

### Beispiel 22

### 2-(3-{[6-Amino-5-(trifluoracetyl)pyridin-2-yl]amino}piperidin-1-yl)-6-(3-chlorphenyl)-4-(trifluormethyl)pyridin-3-carbonitril

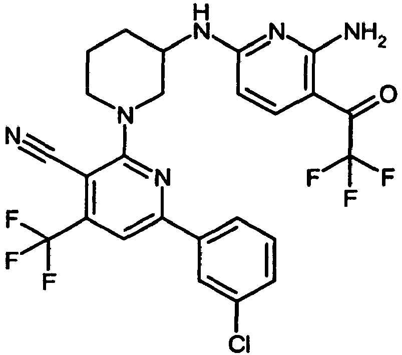

Analog der Herstellung von Beispiel 20 wurden aus 60 mg (0.19 mmol) 2-Chlor-6-(3-chlorphenyl)-4-(trifluormethyl)pyridin-3-carbonitril und 66.8 mg (0.23 mmol) 1-[2-Amino-6-(piperidin-3-ylamino)pyridin-3-yl]-2,2,2-trifluorethanon Hydrochlorid (Beispiel 13A) nach Trennung mittels präparativer HPLC (Methode 13) 96 mg (89% d. Th.) des Produktes als Feststoff erhalten.

LCMS (Methode 8): Rₜ = 1.67 min. (m/z = 569 (M+H)⁺)

¹H-NMR (400MHz, DMSO-d₆): δ = 8.49 (br s, 1H), 8.23 (t, 1H), 8.13 (d, 1H), 7.90 (d, 1H), 7.85 (s, 1H), 7.59 (d, 1H), 7.56 (br s, 1H), 7.51 (d, 1H), 7.46 (m, 1H), 5.89 (d, 1H), 4.22 (m, 1H), 4.13 (d, 1H), 3.9-3.99 (m, 1H), 3.72 (dd, 1H), 3.58-3.67 (m, 1H), 2.0-2.12 (m, 2H), 1.61-1.78 (m, 2H).

Die Enantiomerentrennung von 2-(3-{[6-Amino-5-(trifluoracetyl)pyridin-2-yl]amino}piperidin-1-yl)-6-(3-chlorphenyl)-4-(trifluormethyl)pyridin-3-carbonitril (Beispiel 22) wurde unter den folgenden Bedingungen durchgeführt:
Eine Probe von Beispiel 22 (83 mg) wurde in 20 ml iso-Hexan:Ethanol (4:1) gelöst und über eine Daicel Chiralpak AS-H, 5µm, 250 mm x 20 mm Säule chromatographiert (Fluß: 20 ml/min; Detektion bei 230 nm; Injektionsvolumen: 4500 µl; Eluent: iso-Hexan:Ethanol (80:20), Temperatur: 24°C). Es wurden zwei Fraktionen isoliert:
   **Beispiel Ent-A-22:** Es wurden 26 mg Produkt in > 99% ee isoliert.
   Retentionszeit 4.54 min, spez. Drehwert: [α]²⁰₅₈₉= +328.2° (c = 0.4645g/100 ml Ethanol)
   **Beispiel Ent-B-22:** Es wurden 42 mg Produkt in > 99% ee isoliert.
   Retentionszeit 7.15 min

### Beispiel 23

### 6-(2-Chlorphenyl)-2-{3-[(5-cyanopyridin-2-yl)amino]piperidin-1-yl}-4-(trifluormethyl)pyridin-3-carbonitril

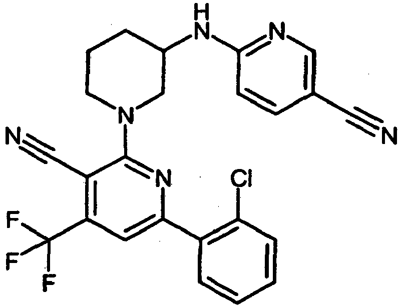

Analog der Herstellung von Beispiel 20 wurden aus 60 mg (0.19 mmol) 2-Chlor-6-(2-chlorphenyl)-4-(trifluormethyl)pyridin-3-carbonitril und 47 mg (0.19 mmol) 6-(Piperidin-3-ylamino)pyridin-3-carbonitril Hydrochlorid (Beispiel 10A) nach Trennung mittels präparativer HPLC (Methode 13) 61 mg (67% d. Th.) des Produktes als Feststoff erhalten.

LCMS (Methode 8): Rₜ = 1.53 min. (m/z = 483 (M+H)⁺)

¹H-NMR (400MHz, DMSO-d₆): δ = 8.31 (d, 1H), 7.67 (dd, 1H), 7.45-7.64 (m, 6H), 6.51 (d, 1H), 4.15 (d, 1H), 3.91-4.07 (m, 2H), 3.52-3.65 (m, 2H), 1.94-2.07 (m, 2H), 1.61-1.75 (m, 2H).

### Beispiel 24

### 2-{3-[(5-Cyanopyridin-2-yl)amino]piperidin-1-yl}-6-(4-fluorphenyl)-4-(trifluormethyl)pyridin-3-carbonitril

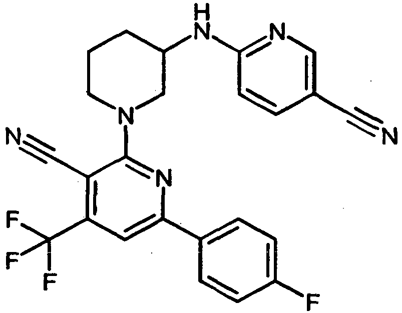

Analog der Herstellung von Beispiel 20 wurden aus 60 mg (0.2 mmol) 2-Chlor-6-(4-fluorphenyl)-4-(trifluormethyl)pyridin-3-carbonitril und 59.5 mg (0.24 mmol) 6-(Piperidin-3-ylamino)pyridin-3-carbonitril Hydrochlorid (Beispiel 10A) nach Trennung mittels präparativer HPLC (Methode 13) 61 mg (66% d. Th.) des Produktes als Feststoff erhalten.

LCMS (Methode 8): Rₜ = 1.5 min. (m/z = 467 (M+H)⁺)

¹H-NMR (400MHz, DMSO-d₆): δ = 8.38 (d, 1H), 8.23-8.29 (m, 2H), 7.76 (s, 1H), 7.57-7.65 (m, 2H), 7.36 (ψt, 2H), 6.52 (d, 1H), 4.19 (dd, 1H), 4.09 (m, 1H), 3.93-4.01 (m, 1H), 3.57-3.71 (m, 2H), 1.92-2.09 (m, 2H), 1.61-1.78 (m, 2H).

### Beispiel 25

### 6-(4-Chlorphenyl)-2- {3-[(5-cyanopyridin-2-yl)amino]piperidin-1-yl}-4-(trifluormethyl)pyridin-3-carbonitril

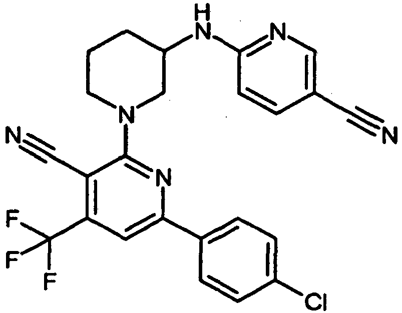

Analog der Herstellung von Beispiel 20 wurden aus 60 mg (0.19 mmol) 2-Chlor-6-(4-chlorphenyl)-4-(trifluormethyl)pyridin-3-carbonitril und 56 mg (0.23 mmol) 6-(Piperidin-3-ylamino)pyridin-3-carbonitril Hydrochlorid (Beispiel 10A) nach Trennung mittels präparativer HPLC (Methode 13) 46 mg (50% d. Th.) des Produktes als Feststoff erhalten.

LCMS (Methode 8): Rₜ = 1.57 min. (m/z = 483 (M+H)⁺)

¹H-NMR (400MHz, DMSO-d₆): δ = 8.38 (d, 1H), 8.21 (d, 2H), 7.78 (s, 1H), 7.55-7.65 (m, 4H), 6.52 (d, 1H), 4.18 (d, 1H), 4.09 (m, 1H), 3.92-4.01 (m, 1H), 3.59-3.72 (m, 2H), 1.96-2.09 (m, 2H), 1.63-1.76 (m, 2H).

### Beispiel 26

### 2-{3-[(6-Amino-5-cyanopyridin-2-yl)amino]piperidin-1-yl}-6-(2-chlorphenyl)-4-(trifluormethyl)-pyridine-3-carbonitril

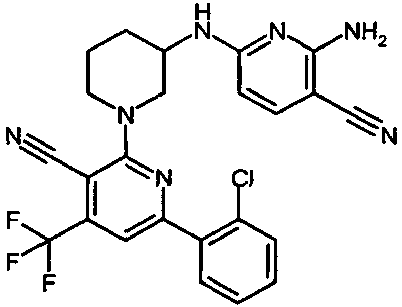

Analog der Herstellung von Beispiel 20 wurden aus 60 mg (0.19 mmol) 2-Chlor-6-(2-chlorphenyl)-4-(trifluormethyl)pyridin-3-carbonitril und 49.5 mg (0.19 mmol) 2-Amino-6-(piperidin-3-ylamino)pyridin-3-carbonitril Hydrochlorid (Beispiel 11A) nach Trennung mittels präparativer HPLC (Methode 13) 94 mg (99% d. Th.) des Produktes als Feststoff erhalten.

LCMS (Methode 3): Rₜ = 2.87 min. (m/z = 498 (M+H)⁺)

¹H-NMR (400MHz, DMSO-d₆): δ = 7.69 (dd, 1H), 7.61 (dd, 1H), 7.54 (dd, 1H), 7.45-7.52 (m, 2H), 7.22 (d, 1H), 7.10 (br s, 1H), 6.21 (s, 2H), 5.75-5.8 (m, 1H), 4.24 (d, 1H), 3.91-4.10 (m, 2H), 3.33-3.45 (m, 2H), 1.9-2.05 (m, 2H), 1.5-1.73 (m, 2H).

Die Enantiomerentrennung von 2-{3-[(6-Amino-5-cyanopyridin-2-yl)amino]piperidin-1-yl}-6-(2-chlorphenyl)-4-(trifluormethyl)pyridine-3-carbonitril (Beispiel 26) wurde unter den folgenden Bedingungen durchgeführt:
Eine Probe von Beispiel 26 (83 mg) wurde in 20 ml iso-Hexan:Ethanol (3:2) gelöst und über eine Daicel Chiralpak AS-H, 5µm, 250 mm x 20 mm Säule chromatographiert (Fluß: 20 ml/min; Detektion bei 230 nm; Injektionsvolumen: 4500 µl; Eluent: iso-Hexan:Ethanol (70:30), Temperatur: 24°C). Es wurden zwei Fraktionen isoliert:
   **Beispiel Ent-A-26:** Es wurden 26 mg Produkt in > 99% ee isoliert.
   Retentionszeit 7.97 min, spez. Drehwert: [α]²⁰₅₈₉= +208° (c = 0.230g/100 ml Ethanol)
   **Beispiel Ent-B-26:** Es wurden 26 mg Produkt in > 99% ee isoliert.
   Retentionszeit 13.57 min

### Beispiel 27

### 2-{3-[(6-Amino-5-cyanopyridin-2-yl)amino]piperidin-1-yl}-6-(4-fluorphenyl)-4-(trifluormethyl)-pyridin-3-carbonitril

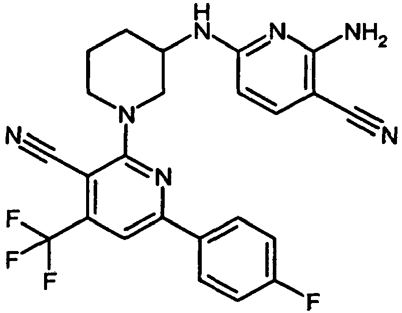

Analog der Herstellung von Beispiel 20 wurden aus 60 mg (0.2 mmol) 2-Chlor-6-(4-fluorphenyl)-4-(trifluormethyl)pyridin-3-carbonitril und 62.6 mg (0.24 mmol) 2-Amino-6-(piperidin-3-ylamino)pyridin-3-carbonitril Hydrochlorid (Beispiel 11A) nach Trennung mittels präparativer HPLC (Methode 13) 90 mg (94% d. Th.) des Produktes als Feststoff erhalten.

LCMS (Methode 3): Rₜ = 2.83 min. (m/z = 482 (M+H)⁺)

¹H-NMR (400MHz, DMSO-d₆): δ = 8.21-8.28 (m, 1H), 7.78 (s, 1H), 7.35 (ψt, 2H), 7.27 (d, 1H), 7.12 (d, 1H), 6.25 (br s, 2H), 5.77 (d, 2H), 4.19 (d, 1H), 3.98-4.1 (d, 2H), 3.42-3.58 (m, 2H), 1.92-2.07 (m, 2H), 1.56-1.78 (m, 2H).

### B) Bewertung der physiologischen Wirksamkeit

Die Eignung der erfindungsgemäßen Verbindungen zur Behandlung von hämatologischen Erkrankungen kann in folgenden Assaysystemen gezeigt werden:

### In vitro Assay

Die inhibitorische Aktivität von Wirksubstanzen wird in einem biochemischen Assay bestimmt. Die dazu benötigten Bestandteile werden in einer schwarzen 384-Loch-Mikrotitterplatte mit transparentem Boden (Firma Greiner, Katalognummer 781092) gemischt. Benötigt werden dabei pro Loch der 384-Loch-Mikrotitterplatte 5 nM GSK3ß (Firma Upstate, Katalognummer 14-306), 40 µM GSK3ß-Substrat GSM (Sequenz H-RRRPASVPPSPSLSRHS-(pS)-HQRR, Firma Upstate, Katalognummer 2-533), 30 µM Nicotinsäureamid-Adenin-Dinucleotid NADH (Roche Diagnostics, Katalognummer 10107735), 50 µM Adenosin-triphoshat ATP (Firma Sigma, Katalognummer A7966), 2 mM Phosphoenolpyruvat (Firma Roche, Katalognummer 128112), sowie ca. 1 U/ml Pyruvatkinase und ca. 1 U/ml Lactatdehydrogenase, die zusammen in einer Stammformulierung vorliegen (Firma Roche, Katalognummer 10737291001, Suspension mit ca. 450 U/ml Pyruvatkinase-Aktivität, ca. 450 U/ml Lactatdehydrogenase-Aktivität in 3.2 mM Ammoniumsulfat-Lösung pH 6). Wobei 1 Unit Pyruvatkinase 1 µmol Phosphoenolpyruvat zu Pyruvat pro Minute bei pH 7.6 und 37°C umsetzt und wobei 1 Unit Laktatdehydrogenase 1 µmol Pyruvat zu Lactat pro Minute bei pH 7.5 und 37°C reduziert. Der benötigte Reaktionspuffer, in dem die biochemische Reaktion abläuft, besteht aus 50 mM Trizma Hydrochlorid Tris- HCl pH: 7.5 (Firma Sigma, Katalognummer T3253), 5 mM Magnesiumchlorid MgCl₂ (Firma Sigma, Katalognummer M8266), 0.2 mM DL-Dithiothreitol DTT (Firma Sigma, Katalognummer D9779), 2 mM Ethylendiaminethertetrasäure EDTA (Firma Sigma, Katalognummer E6758), 0.01% Triton X-100 (Firma Sigma, Katalognummer T8787) und 0.05% Bovines Serumalbumin BSA (Firma Sigma, Katalognummer B4287).

Wirksubstanzen werden in Dimethylsulfoxid DMSO (Firma Sigma, Katalognummer D8418) in einer Konzentration von 10 mM gelöst. Wirksubstanzen werden in Konzentrationsreihen von 10 µM, 1 µM, 0.1 µM, 0.01 µM, 0.001 µM, 0.0001 µM, 0.00001 µM, 0.000001 µM zu den Ansätzen der biochemischen Reaktion zugegeben. Als Kontrolle wird statt Substanz Dimethylsulfoxid in einer Endkonzentration von 0.1 % zugesetzt.

Die Reaktion wird für 2 Stunden bei 30°C inkubiert und anschließend die entstandene Fluoreszenz in einem Tecan Safire-XFLUOR4-Gerät, Version V4.50 (Serienummer 12901300283) unter den Spezifikationen: Messmodus - Fluoreszenz, von unten gemessen, Extinktionswellenlänge 340 nm, Emissionswellenlänge 465 nm, Spaltbreite Extinktion 5 nm, Spaltbreite Emission 5 nm, Verstärkermodus 120, Verzögerung 0 µs, Anzahl Lichtblitze pro Messung 3, und einer Integrationszeit von 40 µs gemessen.

Die Aktivität der GSK3ß wird in Fluoreszenz-Einheiten ermittelt, wobei die Werte von nichtinhibierter Kinase gleich 100% und vollständig inhibierter Kinase gleich 0% gesetzt werden. Die Aktivität der Wirksubstanzen wird auf diese 0% und 100% verrechnet.

Tabelle A zeigt IC₅₀-Werte, die in dem oben beschriebenen Assay ermittelt wurden:

**Tabelle A**

| **Beispiel-Nr.** | **IC₅₀ [nM]** |
|---|---|
| **Ent-B-2** | 5.04 |
| **6** | 26 |
| **7** | 12 |
| **Ent-B-12** | 6.3 |

### CD34+-Proliefrationsassays zur Testung von GSK3ß-Inhibitoren

Adulte hämatopoetische Stammzellen sind durch die spezifische Ausprägung von membranständigen Proteinen gekennzeichnet. Entsprechend ihrem Molekulargewicht sind diese Oberflächenmarker mit einer entsprechenden Nummer versehen. Zu dieser Klasse gehört auch das als CD34 bezeichnete Molekül, welches zur Identifizierung, Charakterisierung und Isolierung von adulten hämtopoetischen Stammzellen dient. Diese Stammzellen können dabei aus dem Knochenmark, dem peripheren Blut oder aus Nabelschnurblut isoliert werden. In in vitro-Kulturen sind diese Zellen begrenzt lebensfähig, können aber durch unterschiedlichste Zusätze zum Kulutrmedium zu Proliferation und Differenzierung angeregt werden. CD34-positive Zellen werden hier verwendet, um den Einfluss von Substanzen auf die Aktivität der Glykogen Synthase Kinase 3 zu testen. Zu diesem Zweck werden in einem ersten Schritt über differentielle Zentrifugationsschritte mononukleare Zellen aus Nabelschnurblut isoliert.

Dazu wird Nabelschnurblut 1:4 mit Phosphat-gepufferter Salzlösung verdünnt. 50 Milliliter Zentrifugationgefäße werden mit 17 Milliliter Ficoll (Dichte 1.077, Ficoll Paque Plus; Pharmacia, Katalognummer 17-1440-02) beschickt. Darauf werden 30 Milliliter des 1:4 verdünnten Nabelschnurblutes aufgeschichtet und anschließend für 30 Minuten bei 400 x g bei Raumtemperatur zentrifuigert. Die Bremsen der Zentrifuge sind dabei ausgeschaltet. Die mononukleären Zellen sammeln sich durch die Zentrifugation in der Interphase. Diese wird mit Hilfe einer 30 Milliliter-Pipette abgenommen und in ein neues 50 Milliliter Zentrifugationsgefäß überführt und das Volumen anschließend mit der Phosphat-gepufferten Salzlösung auf 30 ml ausgefüllt. Diese Zellen werden für 10 Minuten bei Raumtemperatur mit eingeschalteter Bremse bei 300 x g zentrifuigert. Der Überstand wird verworfen und das entstandene Zellpellet in 30 Milliliter Phosphat-gepufferter Salzlösung resuspendiert. Diese Zellen werden erneut für 15 Minuten bei 20 °C bei 200 x g und eingeschalteter Bremse zentrifugiert.

Zur Isolierung der CD34-positiven Zellen aus die angereicherten mononukleären Zellen in einer Konzentration von 1 X 10⁸ Zellen pro 300 Mikroliter MACS-Puffer (0.5% Endotoxin-freies bovines Serumalbumin in Phosphat-gepufferter Salzlösung) resuspendiert. Es erfolgt die Zugabe von 100 Mikrolitern FCR Blocking Reagenz (Miltenyi Biotec, Katalognummer 130-046-702) sowie 100 Mikrolitern an CD34 Micro Beads (Miltenyi Biotec, Katalognummer 130-046-702). Diese Suspension wird für 30 Minuten bei 4°C inkubiert. Anschließend werden die Zellen mit dem 20-fachem Volumen MACS Puffer verdünnen und 10 Minuten bei 300 x g zentrifugiert. Der Überstand wird verworfen und die Zellen in 500 Mikrolitern MACS Puffer resuspendiert. Die so behandelten Zellen werden auf einer LS-Säule (Miltenyi Biotec, katalognummer 130-042-401) aufgetragen und unter Verwendung eines Midi MACS Magneten (Miltenyi Biotec, Katalognummer 130-042-303) aufgereinigt.

Die Anzahl an CD34-positiven Zellen wird über das auszählen der Zellen unter Verwendung einer Neubauer-Kammer durchgeführt. Die Bestimmung der Reinheit der Zellen erfolgt nach Standardprotokollen unter Verwendung der Fluorescent Activated Cell Sorting -Methode (Becton Dickinson, BD FACS™ Sample Prep Assistant SPAII Upgrade Kit, Katalognummer 337642).

Zur Bestimmung des Einflusses einer Modulation der GSK3-Aktivität werden CD34-positive Zellen über 7 Tage in einer 96-Loch-Mikrotitterplatte bei 37°C und 5% Kohlendioxid inkubiert und anschließend die Proliferationsraten anhand der Zellzahlen bestimmt.

Zu diesem Zweck werden 5000 CD34-positive Zellen pro Loch einer 96-U-Boden-Loch-Mikrotitterplatte (Greiner Bio-One, Katalognummer 650 180) in 100 Mikroliter IMDM-Medium (Life Technology, Katalognummer 12440-046), 10% fetales Kälberserum (Life Technology, Katalognummer 10082-139) und 20 Nanogramm pro Milliliter Stem Cell Factor (R&D, Katalognummer 255-SC-010) aufgenommen. Zusätzlich werden die Zellen noch unterschiedlichen Konzentrationen an mit Dimethylsulfoxid (Sigma Aldrich, Katalognummer D5879-1L) gelösten Substanzen versetzt. Dabei werden jeweils 4 Löcher mit der angegebenen Zellzahl von 5000 CD34-positiven Zellen pro Loch mit 10 Mikromol, 4 Löcher mit 5 Mikromol, 4 Löcher mit 2.5 Mikromol, 4 Löcher mit 1.25 Mikromol, 4 Löcher mit 0.625 Mikromol, 4 Löcher mit 0.3125 Mikromol, 4 Löcher mit 0.156 Mikromol, 4 Löcher mit 0.078 Mikromol und als Kontrolle 4 Löcher mit 0.1% Dimethylsulfoxid als Endkonzentration versehen.

Diese so behandelten Zellen werden für 7 Tage in einem Zellkultur-Brutschrank bei 37°C und 5% Kohlendioxid inkubiert. Durch erneutes zählen der Zellen unter Verwendung einer Neubauer-Zählkammer wird die Proliferationsrate bestimmt, wobei die nur mit dem Stem Cell Factor versehenen Zellen als 100%-Wert gesetzt und alle anderen Werte auf diesen Wert bezogen sind.

### In-vivo Assay

Die Untersuchungen der in vivo-Wirkung der erfindungsmäßigen Verbindungen erfolgt unter Verwendung von 6 Wochen alten, 18 - 22 g schweren, männlichen C57BL/6-Mäusen (Charles River, Sulzfeld, Deutschland). Diese Tiere werden artgerecht mit 12-stündigen Licht- und Dunkelzyklen unter konstanten klimatischen Bedingungen gehalten und mit Wasser und Mausfutter *ad libitum* ernährt. Die Konzentrationen an verwendeten Chemotherapeutika werden den Tieren gemäß den Angaben der Hersteller mittels intra-peritonealen (i.p.) Injektionen im kaudalen Drittel des Bauches verabreicht. Gleichermaßen wird mit den erfindungsrelevanten Substanzen verfahren. Blutabnahmen erfolgen mit Hilfe von Pasteur-Pipetten aus dem retrobulbären Venenplexus. Die Bestimmung der Anzahl neutrophiler Granulozyten erfolgt vollautomatisch unter Verwendung von Durchflußzytometriesystemen.

### CYP-Inhibitionstest

Die Fähigkeit von Substanzen, CYP1A2, CYP 2C8, CYP2C9, CYP2D6 und CYP3A4 im Menschen inhibieren zu können, wird untersucht mit gepoolten Human-Lebermikrosomen als Enzymquelle in Gegenwart von Standardsubstraten (s.u.), die CYP-Isoform-spezifische Metaboliten bilden. Die Inhibitionseffekte werden bei sechs verschiedenen Konzentrationen der Testverbindungen untersucht (1.5, 3.1, 6.3, 12.5, 25 sowie 50 µM), mit dem Ausmaß der CYP-Isoform-spezifischen Metabolitenbildung der Standardsubstrate in Abwesenheit der Testverbindungen verglichen und die entsprechenden IC₅₀-Werte berechnet. Ein Standard-Inhibitor, der eine einzelne CYP-Isoform spezifisch inhibiert, dient als Kontrolle der erhaltenen Ergebnisse.

### Durchführung:

Die Inkubation von Phenacetin, Amodiaquin, Diclofenac, Dextromethorphan oder Midazolam mit Human-Lebermikrosomen in Gegenwart von jeweils sechs verschiedenen Konzentrationen einer Testverbindung (als potentiellem Inhibitor) wird auf einer Workstation durchgeführt (Tecan, Genesis, Crailsheim, Deutschland). Standard-Inkubationsgemische enthalten 1.3 mM NADP, 3.3 mM MgCl₂ x 6 H₂O, 3.3 mM Glukose-6-phosphat, Glukose-6-phosphat-Dehydrogenase (0.4 U/ml) und 100 mM Phosphat-Puffer (pH 7.4) in einem Gesamtvolumen von 200 µl. Testverbindungen werden bevorzugt in Acetonitril gelöst. 96-Lochplatten werden eine definierte Zeit bei 37°C mit gepoolten Human-Lebermikrosomen inkubiert. Die Reaktionen werden durch Zugabe von 100 µl Acetonitril, worin sich ein geeigneter interner Standard befindet, abgestoppt. Gefällte Proteine werden durch Zentrifugation abgetrennt, die Überstände werden vereinigt und mittels LC-MS/MS analysiert.

### Bestimmung der Löslichkeit

Benötigte Reagenzien:
- PBS-Puffer pH 6.5: 61.86 g Natriumchlorid p.a. (z.B. Fa. Merck, Art.-Nr. 1.06404.1000), 39.54 g Natriumdihydrogenphosphat p.a. (z.B. Fa. Merck, Art.-Nr. 1.06346.1000) und 83.35 g 1 N Natriumhydroxid-Lösung (z.B. Fa. Bernd Kraft GmbH, Art.-Nr. 01030.4000) in einen 1 Liter-Messkolben einwiegen, mit Wasser auffüllen und ca. 1 Stunde rühren. Von dieser Lösung 500 ml in einen 5 Liter-Messkolben geben und mit Wasser auffüllen. Mit 1 N Natriumhydroxid-Lösung auf pH 6.5 einstellen.
- Dimethylsulfoxid (z.B. Fa. Baker, Art.-Nr. 7157.2500)
- destilliertes Wasser
- Acetonitril Chromasolv (z.B. Riedel-de Haen Art. Nr. 34851)
- 50%ige Ameisensäure p.a. (z.B. Fluka Art. Nr. 09676)

### Herstellung der Ausgangslösung:

Mindestens 1.5 mg der Testsubstanz werden in ein Wide Mouth 10mm Screw V-Vial (Fa. Glastechnik Gräfenroda GmbH, Art.-Nr. 8004-WM-H/V15µ) mit passender Schraubkappe und Septum genau eingewogen, mit Dimethylsulfoxid zu einer Konzentration von 50 mg/ml versetzt und 30 Minuten mittels eines Vortexers geschüttelt.

### Herstellung der Kalibrierlösungen:

Die notwendigen Pipettierschritte erfolgen in 1.2 ml 96er Deep Well Plate (DWP) (z.B. HJ-Bioanalytik GmbH Art.-Nr. 850289) mittels eines Liquid-Handling-Roboters. Als Lösemittel wird ein Gemisch aus Acetonitril Chromasolv / destilliertem Wasser 8:2 verwendet.

*Herstellung der Ausgangslösung für Kalibrierlösungen (Stammlösung):* 10 µl der Ausgangslösung werden mit 833 µl des Lösemittelgemisch versetzt (Konzentration = 600 µg/ml) und homogenisiert. Es werden von jeder Testsubstanz zwei 1:100 Verdünnungen in separaten DWP's hergestellt und wiederum homogenisiert. Eine der 1:100 Verdünnungen wird für die Herstellung der Kalibrierlösungen verwendet, die zweite Verdünnung wird für die Optimierung der MS/MS-Parameter verwendet.

*Kalibrierlösung 5 (600 ng*/*ml):* 30 µl der Stammlösung werden mit 270 µl Lösemittelgemisch versetzt und homogenisiert.

*Kalibrierlösung 4 (60 ng*/*ml):* 30 µl der Kalibrierlösung 5 werden mit 270 µl Lösemittelgemisch versetzt und homogenisiert.

*Kalibrierlösung 3 (12 ng*/*ml):* 100 µl der Kalibrierlösung 4 werden mit 400 µl Lösemittelgemisch versetzt und homogenisiert.

*Kalibrierlösung 2 (1.2 ng*/*ml):* 30 µl der Kalibrierlösung 3 werden mit 270 µl Lösemittelgemisch versetzt und homogenisiert.

*Kalibrierlösung 1 (0.6 ng*/*ml):* 150 µl der Kalibrierlösung 2 werden mit 150 µl Lösemittelgemisch versetzt und homogenisiert.

### Herstellung der Probenlösungen:

Die notwendigen Pipettierschritte erfolgen in 1.2 ml 96er DWP (z.B. HJ-Bioanalytik GmbH Art.-Nr. 850289) mittels eines Liquid-Handling-Roboters.

10.1 µl der Stammlösung werden mit 1000 µl PBS-Puffer pH 6.5 versetzt.

### Durchführung:

Die notwendigen Pipettierschritte erfolgen in 1.2 ml 96er DWP (z.B. HJ-Bioanalytik GmbH Art.-Nr. 850289) mittels eines Liquid-Handling-Roboters.

Die so hergestellten Probenlösungen werden 24 Stunden bei 1400 rpm mittels eines temperierbaren Schüttlers (z.B. Fa. Eppendorf Thermomixer comfort Art.-Nr. 5355 000.011) bei 20°C geschüttelt. Von diesen Lösungen werden jeweils 180 µl abgenommen und in Beckman Polyallomer Centrifuge Tubes (Art.-Nr. 343621) überführt. Diese Lösungen werden 1 Stunde mit ca. 223.000 x g zentrifugiert (z.B. Fa. Beckman Optima L-90K Ultracentrifuge mit Type 42.2 Ti Rotor bei 42.000 rpm). Von jeder Probenlösung werden 100 µl des Überstandes abgenommen und 1:10 und 1:1000 mit PBS-Puffer 6.5 verdünnt.

### Analytik:

Die Proben werden mittels HPLC/MS-MS analysiert. Quantifiziert wird über eine Fünf-Punkt-Kalibrationskurve der Testverbindung. Die Löslichkeit wird in mg/l ausgedrückt. Analysensequenz: 1) Blank (Lösemittelgemisch); 2) Kalibrierlösung 0.6 ng/ml; 3) Kalibrierlösung 1.2 ng/ml; 4) Kalibrierlösung 12 ng/ml; 5) Kalibrierlösung 60 ng/ml; 6) Kalibrierlösung 600 ng/ml; 7) Blank (Lösemittelgemisch); 8) Probenlösung 1:1000; 7) Probenlösung 1:10.

### HPLC/MS-MS Methode

HPLC: Agilent 1100 , quat. Pumpe (G1311A), Autosampler CTC HTS PAL, Degaser (G1322A) und Säulenthermostat (G1316A); Säule: Oasis HLB 20 mm x 2.1 mm, 25 µ; Temperatur: 40°C; Eluent A: Wasser + 0.5 ml Ameisensäure/l; Eluent B: Acetonitril + 0.5 ml Ameisensäure/l; Flussrate: 2.5 ml/min; Stoptime 1.5 min; Gradient: 0 min 95% A, 5% B; Rampe: 0-0.5 min 5% A, 95% B; 0.5-0.84 min 5% A, 95% B; Rampe: 0.84-0.85 min 95% A, 5% B; 0.85-1.5 min 95% A, 5% B.

MS/MS:WATERS Quattro Micro Tandem MS/MS; Z-Spray API-Interface; HPLC-MS-Eingangssplitter 1:20; Messung im ESI-Mode

Die Geräteparameter werden für jede Testsubstanz durch Injektion der weiter oben beschriebenen Stammlösung (zweite 1:100 Verdünnung) mittels der MassLynx/QuanOptimize-Software automatisch optimiert.

### C) Ausführungsbeispiele für pharmazeutische Zusammensetzungen

Die erfindungsgemäßen Substanzen können folgendermaßen in pharmazeutische Zubereitungen überführt werden:

### Tablette:

### Zusammensetzune:

100 mg der Verbindung des Beispiels 1, 50 mg Lactose (Monohydrat), 50 mg Maisstärke, 10 mg Polyvinylpyrolidon (PVP 25) (Fa. BASF, Deutschland) und 2 mg Magnesiumstearat.

Tablettengewicht 212 mg. Durchmesser 8 mm, Wölbungsradius 12 mm.

### Herstellung:

Die Mischung aus der Verbindung des Beispiels 1, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben).

### Orale Suspension:

### Zusammensetzung:

1000 mg der Verbindung des Beispiels 1, 1000 mg Ethanol (96%), 400 mg Rhodigel (Xanthan gum) (Fa. FMC, USA) und 99 g Wasser.

Einer Einzeldosis von 100 mg der erfindungsgemäßen Verbindung entsprechen 10 ml orale Suspension.

### Herstellung

Das Rhodigel wird in Ethanol suspendiert, die Verbindung des Beispiels 1 wird der Suspension zugefügt. Unter Rühren erfolgt die Zugabe des Wassers. Bis zum Abschluss der Quellung des Rhodigels wird ca. 6h gerührt.

### Intravenös applizierbare Lösung:

### Zusammensetzung:

1 mg der Verbindung von Beispiel 1, 15 g Polyethylenglykol 400 und 250 g Wasser für Injektionszwecke.

### Herstellung:

Die Verbindung von Beispiel 1 wird zusammen mit Polyethylenglykol 400 in dem Wasser unter Rühren gelöst. Die Lösung wird sterilfiltriert (Porendurchmesser 0.22 µm) und unter aseptischen Bedingungen in hitzesterilisierte Infusionsflaschen abgefüllt. Diese werden mit Infusionsstopfen und Bördelkappen verschlossen.

## Patentansprüche

1. Verbindung der Formel 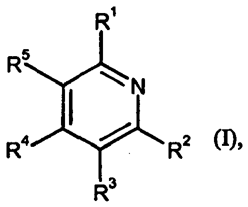 in welcher
R¹ für eine Gruppe der Formel 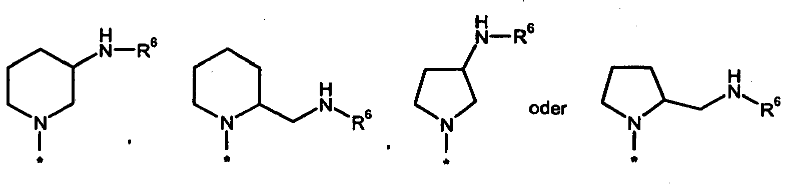 steht,
wobei
* die Anknüpfstelle an den Heterocyclus bedeutet,
R⁶ für Pyrid-2-yl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,2-Pyrazol-5-yl steht,
wobei Pyrid-2-yl, Pyrimid-2-yl, 1,3-Thiazol-2-yl und 1,3-Thiazol-4-yl substituiert sind mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkylamino, Alkylcarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
und
wobei 2-Aminopyrimid-4-yl, 2-Cyclopropylaminopyrimid-4-yl, 2-Methylamino-pyrimid-4-yl, 2-Ethylaminopyrimid-4-yl und 1,2-Pyrazol-5-yl substituiert sein können mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
R² für Phenyl steht,
wobei Phenyl substituiert sein kann mit 1 bis 3 Substituenten, wobei die Substituenten unabhängig voneinander ausgewählt werden aus der Gruppe bestehend aus Hydroxy, Halogen, Cyano, Trifluormethyl, Trifluormethoxy, Aminocarbonyl, C₁-C₄-Alkyl, C₁-C₄-Alkoxy, C₁-C₄-Alkylamino, C₁-C₄-Alkoxymethyl, C₁-C₄-Alkylaminomethyl, C₁-C₄-Alkylcarbonyl, C₁-C₄-Alkoxycarbonyl, C₁-C₄-Alkylaminocarbonyl, C₁-C₄-Alkylcarbonylamino, C₁-C₄-Alkylsulfonyl, C₁-C₄-Alkylsulfonylamino, C₁-C₄-Alkylaminosulfonyl, Phenyl, Pyrrolidinyl, Piperidinyl, Morpholinyl, Piperazinyl, Pyrrolidinylmethyl, Piperidinylmethyl, Morpholinylmethyl und Piperazinylmethyl,
worin Phenyl, Pyrrolidinyl, Piperidinyl, Morpholinyl, Piperazinyl, Pyrrolidinylmethyl, Piperidinylmethyl, Morpholinylmethyl und Piperazinylmethyl substituiert sein können mit 1 bis 3 Substituenten, wobei die Substituenten unabhängig voneinander ausgewählt werden aus der Gruppe bestehend aus Halogen, Cyano, Trifluormethyl, Trifluormethoxy und C₁-C₄-Alkyl,
R³ für Wasserstoff, Halogen, Cyano, Trifluormethyl, C₁-C₃-Alkyl oder Cyclopropyl steht,
entweder
R⁴ für Wasserstoff, Halogen, Cyano, Trifluormethyl, C₁-C₃-Alkyl oder Cyclopropyl steht,
und
R⁵ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
oder
R⁴ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
R⁵ für Wasserstoff, Halogen, Cyano, Trifluormethyl, C₁-C₃-Alkyl oder Cyclopropyl steht,
oder eines ihrer Salze, ihrer Solvate oder der Solvate ihrer Salze.

2. Verbindung nach Anspruch 1, **dadurch gekennzeichnet, dass**
R¹ für eine Gruppe der Formel 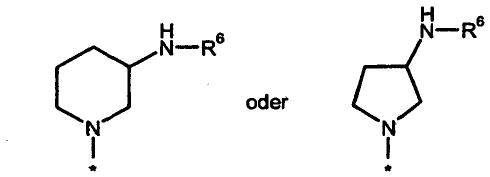 steht,
wobei
* die Anknüpfstelle an den Heterocyclus bedeutet,
R⁶ für Pyrid-2-yl, Pyrimid-2-yl, 2-Aminopyrimid-4-yl, 2-Cyclopropylaminopyrimid-4-yl, 2-Methylaminopyrimid-4-yl, 2-Ethylaminopyrimid-4-yl, 1,3-Thiazol-2-yl, 1,3-Thiazol-4-yl oder 1,2-Pyrazol-5-yl steht,
wobei Pyrid-2-yl, Pyrimid-2-yl, 1,3-Thiazol-2-yl und 1,3-Thiazol-4-yl substituiert sind mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
worin Alkyl, Alkylamino, Alkylcarbonyl, Alkylaminocarbonyl und Cycloalkylcarbonyl substituiert sein können mit einem Substituenten, wobei der Substituent ausgewählt wird aus der Gruppe bestehend aus Halogen, Cyano, Hydroxy, Amino, Trifluormethyl und C₃-C₆-Cycloalkyl,
und
wobei 2-Aminopyrimid-4-yl, 2-Cyclopropylaminopyrimid-4-yl, 2-Methylamino-pyrimid-4-yl, 2-Ethylaminopyrimid-4-yl und 1,2-Pyrazol-5-yl substituiert sein können mit 1 bis 2 Substituenten, wobei die Substituenten unabhängig voneinander ausgewählt werden aus der Gruppe bestehend aus Halogen, Cyano, Nitro, Amino, Trifluormethyl, Trifluormethoxy, Aminocarbonyl, Trifluormethylcarbonyl, C₁-C₄-Alkyl, C₁-C₄-Alkoxy, C₁-C₄-Alkylamino, C₁-C₄-Alkylcarbonyl, C₁-C₄-Alkoxycarbonyl, C₁-C₄-Alkylaminocarbonyl und C₃-C₆-Cycloalkylcarbonyl,
R² für Phenyl steht,
wobei Phenyl substituiert sein kann mit 1 bis 3 Substituenten, wobei die Substituenten unabhängig voneinander ausgewählt werden aus der Gruppe bestehend aus Chlor, Fluor, Trifluormethyl, Trifluormethoxy, Methyl und Methoxy,
R³ für Wasserstoff steht,
entweder
R⁴ für Wasserstoff steht,
und
R⁵ für Halogen, Cyano, Trifluormethyl, Hydroxycarbonyl, Aminocarbonyl, C₁-C₄-Alkyl, C₁-C₄-Alkoxycarbonyl, C₁-C₄-Alkylaminocarbonyl oder 5- oder 6-gliedriges Heterocyclylcarbonyl steht,
wobei Heterocyclylcarbonyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkylaminocarbonyl substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
oder
R⁴ für Trifluormethyl steht,
und
R⁵ für Cyano steht,
oder eines ihrer Salze, ihrer Solvate oder der Solvate ihrer Salze.

3. Verbindung nach einem der Ansprüche 1 oder 2, **dadurch gekennzeichnet, dass**
R¹ für eine Gruppe der Formel 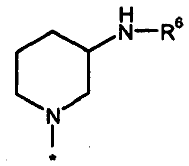 steht,
wobei
* die Anknüpfstelle an den Heterocyclus bedeutet,
R⁶ für Pyrid-2-yl oder 1,3-Thiazol-2-yl steht,
wobei Pyrid-2-yl und 1,3-Thiazol-2-yl substituiert sind mit 1 bis 2 Substituenten, wobei die Substituenten unabhängig voneinander ausgewählt werden aus der Gruppe bestehend aus Cyano, Nitro, Amino, Trifluormethylcarbonyl, Ethylcarbonyl und Methylcarbonyl,
R² für Phenyl steht,
wobei Phenyl substituiert ist mit 1 bis 2 Substituenten, wobei die Substituenten unabhängig voneinander ausgewählt werden aus der Gruppe bestehend aus Chlor, Fluor, Trifluormethyl, Trifluormethoxy, Methyl und Methoxy,
R³ für Wasserstoff steht,
entweder
R⁴ für Wasserstoff steht,
und
R⁵ für Cyano, Trifluormethyl, Hydroxycarbonyl, Methoxycarbonyl, Ethoxycarbonyl, C₁-C₂-Alkylaminocarbonyl, Piperazinylcarbonyl, Piperidinylcarbonyl oder Mopholinylcarbonyl steht,
wobei Piperazinylcarbonyl, Piperidinylcarbonyl und Mopholinylcarbonyl substituiert sein können mit 1 bis 3 Substituenten, wobei die Substituenten unabhängig voneinander ausgewählt werden aus der Gruppe bestehend aus Oxo, Halogen und C₁-C₄-Alkyl,
und
wobei Alkylaminocarbonyl substituiert sein kann mit einem Substituenten, wobei der Substituent ausgewählt wird aus der Gruppe bestehend aus Piperazinyl, Piperidinyl und Mopholinyl,
worin Piperazinyl, Piperidinyl und Mopholinyl substituiert sein können mit 1 bis 2 Substituenten, wobei die Substituenten unabhängig voneinander ausgewählt werden aus der Gruppe bestehend aus Oxo, Halogen und C₁-C₄-Alkyl,
oder
R⁴ für Trifluormethyl steht,
und
R⁵ für Cyano steht,
oder eines ihrer Salze, ihrer Solvate oder der Solvate ihrer Salze.

4. Verbindung nach einem der Ansprüche 1, 2 oder 3, **dadurch gekennzeichnet, dass**
R¹ für eine Gruppe der Formel 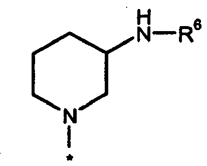 steht,
wobei
* die Anknüpfstelle an den Heterocyclus bedeutet,
R⁶ für eine Gruppe der Formel 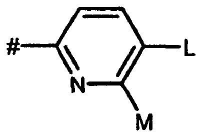 steht,
wobei
# die Anknüpfstelle an NH bedeutet,
entweder
L für Cyano steht,
und
M für Wasserstoff steht,
oder
L für Cyano, Nitro oder Trifluormethylcarbonyl steht,
und
M für Amino steht,
R² für Phenyl steht,
wobei Phenyl substituiert ist mit 1 bis 2 Substituenten, wobei die Substituenten unabhängig voneinander ausgewählt werden aus der Gruppe bestehend aus Chlor, Fluor und Methoxy,
R³ für Wasserstoff steht,
entweder
R⁴ für Wasserstoff steht,
und
R⁵ für Cyano, Hydroxycarbonyl, Methoxycarbonyl, Methylaminocarbonyl, Dimethylaminocarbonyl, Piperazinylcarbonyl oder Piperidinylcarbonyl steht,
wobei Piperazinylcarbonyl und Piperidinylcarbonyl substituiert sind mit 1 bis 2 Substituenten, wobei die Substituenten unabhängig voneinander ausgewählt werden aus der Gruppe bestehend aus Halogen und C₁-C₄-Alkyl,
und
wobei Methylaminocarbonyl und Dimethylaminocarbonyl substituiert sind mit einem Substituenten Piperidinyl,
worin Piperidinyl substituiert ist mit 1 bis 2 Substituenten, wobei die Substituenten unabhängig voneinander ausgewählt werden aus der Gruppe bestehend aus Halogen und C₁-C₄-Alkyl,
oder
R⁴ für Trifluormethyl steht,
und
R⁵ für Cyano steht,
oder eines ihrer Salze, ihrer Solvate oder der Solvate ihrer Salze.

5. Verfahren zur Herstellung einer Verbindung der Formel (I) oder eines ihrer Salze, ihrer Solvate oder der Solvate ihrer Salze nach Anspruch 1, **dadurch gekennzeichnet, dass** entweder
[A] eine Verbindung der Formel 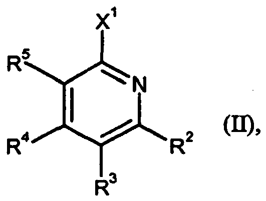 in welcher
R², R³, R⁴ und R⁵ die in Anspruch 1 angegebene Bedeutung haben, und
X¹ für Halogen, bevorzugt Chlor oder Fluor, steht,
mit einer Verbindung der Formel
R¹-H (III),
in welcher
R¹ die in Anspruch 1 angegebene Bedeutung hat,
umgesetzt wird,
oder
[B] eine Verbindung der Formel 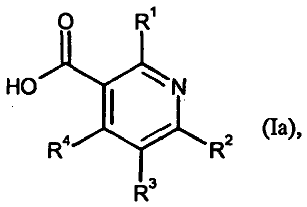 in welcher
R¹, R², R³ und R⁴ die in Anspruch 1 angegebene Bedeutung haben,
mit einer Verbindung der Formel
R^{5a}-H (IV),
in welcher
R^{5a} für C₁-C₄-Alkoxy, C₁-C₄-Alkylamino oder 5- oder 6-gliedriges Heterocyclyl steht,
wobei Heterocyclyl substituiert sein kann mit 1 bis 3 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
und
wobei Alkylamino substituiert sein kann mit einem Substituenten, wobei der Substituent ausgewählt wird aus der Gruppe bestehend aus Hydroxy, Amino, Hydroxycarbonyl, Aminocarbonyl, C₁-C₄-Alkoxy, C₁-C₄-Alkylamino und 5- oder 6-gliedriges Heterocyclyl,
worin Heterocyclyl substituiert sein kann mit 1 bis 2 Substituenten, wobei die Substituenten unabhängig voneinander ausgewählt werden aus der Gruppe bestehend aus Oxo, Halogen, C₁-C₄-Alkyl, C₁-C₄-Alkoxy, C₁-C₄-Alkylamino, C₁-C₄-Alkylcarbonyl, C₁-C₄-Alkoxycarbonyl und C₁-C₄-Alkylaminocarbonyl,
zu einer Verbindung der Formel 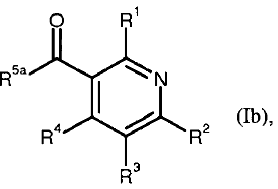 umgesetzt wird.

6. Verbindung nach einem der Ansprüche 1 bis 4 zur Behandlung und/oder Prophylaxe von Krankheiten.

7. Verwendung einer Verbindung nach einem der Ansprüche 1 bis 4 zur Herstellung eines Arzneimittels zur Behandlung und/oder Prophylaxe von Krankheiten.

8. Verwendung einer Verbindung nach einem der Ansprüche 1 bis 4 zur Herstellung eines Arzneimittels zur Behandlung und/oder Prophylaxe von hämatologischen Erkrankungen.

9. Arzneimittel enthaltend eine Verbindung nach einem der Ansprüche 1 bis 4 in Kombination mit einem inerten, nichttoxischen, pharmazeutisch geeigneten Hilfsstoff.

10. Arzneimittel nach Anspruch 9 zur Behandlung und/oder Prophylaxe von hämatologischen Erkrankungen.

## Claims

1. Compound of the formula 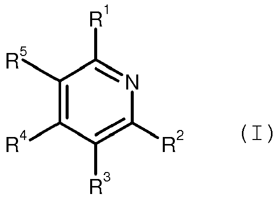 in which
R¹ is a group of the formula 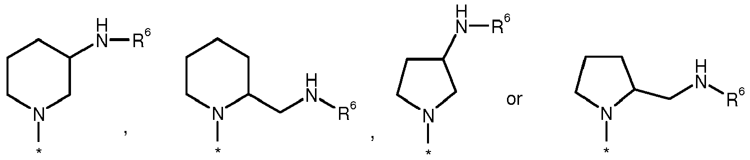 where
* is the attachment site to the heterocycle,
R⁶ is pyrid-2-yl, pyrimid-2-yl, 2-aminopyrimid-4-yl, 2-cyclopropylaminopyrimid-4-yl, 2-methylaminopyrimid-4-yl, 2-ethylaminopyrimid-4-yl, 1,3-thiazol-2-yl, 1,3-thiazol-4-yl or 1,2-pyrazol-5-yl,
where pyrid-2-yl, pyrimid-2-yl, 1,3-thiazol-2-yl and 1,3-thiazol-4-yl are substituted by 1 to 2 substituents, where the substituents are each independently selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl,
in which alkyl, alkylamino, alkylcarbonyl, alkylaminocarbonyl and cycloalkylcarbonyl may be substituted by one substituent, where the substituent is selected from the group consisting of halogen, cyano, hydroxyl, amino, trifluoromethyl and C₃-C₆-cycloalkyl,
and
where 2-aminopyrimid-4-yl, 2-cyclopropylaminopyrimid-4-yl, 2-methylaminopyrimid-4-yl, 2-ethylaminopyrimid-4-yl and 1,2-pyrazol-5-yl may each be substituted by 1 to 2 substituents, where the substituents are each independently selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl,
R² is phenyl,
where phenyl may be substituted by 1 to 3 substituents, where the substituents are each independently selected from the group consisting of hydroxyl, halogen, cyano, trifluoromethyl, trifluoromethoxy, aminocarbonyl, C₁-C₄-alkyl, C₁-C₄-alkoxy, C₁-C₄-alkylamino, C₁-C₄-alkoxymethyl, C₁-C₄-alkylaminomethyl, C₁-C₄-alkylcarbonyl, C₁-C₄-alkoxycarbonyl, C₁-C₄-alkylaminocarbonyl, C₁-C₄-alkylcarbonylamino, C₁-C₄-alkylsulphonyl, C₁-C₄-alkylsulphonylamino, C₁-C₄-alkylaminosulphonyl, phenyl, pyrrolidinyl, piperidinyl, morpholinyl, piperazinyl, pyrrolidinylmethyl, piperidinylmethyl, morpholinylmethyl and piperazinylmethyl,
in which phenyl, pyrrolidinyl, piperidinyl, morpholinyl, piperazinyl, pyrrolidinylmethyl, piperidinylmethyl, morpholinylmethyl and piperazinylmethyl may each be substituted by 1 to 3 substituents, where the substituents are each independently selected from the group consisting of halogen, cyano, trifluoromethyl, trifluoromethoxy and C₁-C₄-alkyl,
R³ is hydrogen, halogen, cyano, trifluoromethyl, C₁-C₃-alkyl or cyclopropyl,
either
R⁴ is hydrogen, halogen, cyano, trifluoromethyl, C₁-C₃-alkyl or cyclopropyl,
and
R⁵ is halogen, cyano, trifluoromethyl, hydroxycarbonyl, aminocarbonyl, C₁-C₄-alkyl, C₁-C₄-alkoxycarbonyl, C₁-C₄-alkylaminocarbonyl or 5- or 6-membered heterocyclylcarbonyl, where heterocyclylcarbonyl may be substituted by 1 to 3 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkylaminocarbonyl may be substituted by one substituent, where the substituent is selected from the group consisting of hydroxyl, amino, hydroxycarbonyl, aminocarbonyl, C₁-C₄-alkoxy, C₁-C₄-alkylamino and 5- or 6-membered heterocyclyl,
in which heterocyclyl may be substituted by 1 to 2 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
or
R⁴ is halogen, cyano, trifluoromethyl, hydroxycarbonyl, aminocarbonyl, C₁-C₄-alkyl, C₁-C₄-alkoxycarbonyl, C₁-C₄-alkylaminocarbonyl or 5- or 6-membered heterocyclylcarbonyl, where heterocyclylcarbonyl may be substituted by 1 to 3 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkylaminocarbonyl may be substituted by one substituent, where the substituent is selected from the group consisting of hydroxyl, amino, hydroxycarbonyl, aminocarbonyl, C₁-C₄-alkoxy, C₁-C₄-alkylamino and 5- or 6-membered heterocyclyl,
in which heterocyclyl may be substituted by 1 to 2 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
R⁵ is hydrogen, halogen, cyano, trifluoromethyl, C₁-C₃-alkyl or cyclopropyl,
or one of the salts thereof, solvates thereof or solvates of the salts thereof.

2. Compound according to Claim 1, **characterized in that**
R¹ is a group of the formula 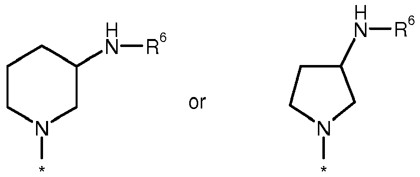 where
* is the attachment site to the heterocycle,
R⁶ is pyrid-2-yl, pyrimid-2-yl, 2-aminopyrimid-4-yl, 2-cyclopropylaminopyrimid-4-yl, 2-methylaminopyrimid-4-yl, 2-ethylaminopyrimid-4-yl, 1,3-thiazol-2-yl, 1,3-thiazol-4-yl or 1,2-pyrazol-5-yl,
where pyrid-2-yl, pyrimid-2-yl, 1,3-thiazol-2-yl and 1,3-thiazol-4-yl are substituted by 1 to 2 substituents, where the substituents are each independently selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl,
in which alkyl, alkylamino, alkylcarbonyl, alkylaminocarbonyl and cycloalkylcarbonyl may be substituted by one substituent, where the substituent is selected from the group consisting of halogen, cyano, hydroxyl, amino, trifluoromethyl and C₃-C₆-cycloalkyl,
and
where 2-aminopyrimid-4-yl, 2-cyclopropylaminopyrimid-4-yl, 2-methylaminopyrimid-4-yl, 2-ethylaminopyrimid-4-yl and 1,2-pyrazol-5-yl may each be substituted by 1 to 2 substituents, where the substituents are each independently selected from the group consisting of halogen, cyano, nitro, amino, trifluoromethyl, trifluoromethoxy, aminocarbonyl, trifluoromethylcarbonyl, C₁-C₄-alkyl, C₁-C₄-alkoxy, C₁-C₄-alkylamino, C₁-C₄-alkylcarbonyl, C₁-C₄-alkoxycarbonyl, C₁-C₄-alkylaminocarbonyl and C₃-C₆-cycloalkylcarbonyl,
R² is phenyl,
where phenyl may be substituted by 1 to 3 substituents, where the substituents are each independently selected from the group consisting of chlorine, fluorine, trifluoromethyl, trifluoromethoxy, methyl and methoxy,
R³ is hydrogen,
either
R⁴ is hydrogen,
and
R⁵ is halogen, cyano, trifluoromethyl, hydroxycarbonyl, aminocarbonyl, C₁-C₄-alkyl, C₁-C₄-alkoxycarbonyl, C₁-C₄-alkylaminocarbonyl or 5- or 6-membered heterocyclylcarbonyl, where heterocyclylcarbonyl may be substituted by 1 to 3 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkylaminocarbonyl may be substituted by one substituent, where the substituent is selected from the group consisting of hydroxyl, amino, hydroxycarbonyl, aminocarbonyl, C₁-C₄-alkoxy, C₁-C₄-alkylamino and 5- or 6-membered heterocyclyl,
in which heterocyclyl may be substituted by 1 to 2 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
or
R⁴ is trifluoromethyl,
and
R⁵ is cyano,
or one of the salts thereof, solvates thereof or solvates of the salts thereof.

3. Compound according to either of Claims 1 and 2, **characterized in that**
R¹ is a group of the formula 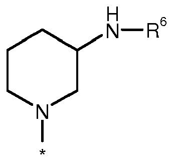 where
* is the attachment site to the heterocycle,
R⁶ is pyrid-2-yl or 1,3-thiazol-2-yl,
where pyrid-2-yl and 1,3-thiazol-2-yl are substituted by 1 to 2 substituents, where the substituents are each independently selected from the group consisting of cyano, nitro, amino, trifluoromethylcarbonyl, ethylcarbonyl and methylcarbonyl,
R² is phenyl,
where phenyl is substituted by 1 or 2 substituents, where the substituents are each independently selected from the group consisting of chlorine, fluorine, trifluoromethyl, trifluoromethoxy, methyl and methoxy,
R³ is hydrogen,
either
R⁴ is hydrogen,
and
R⁵ is cyano, trifluoromethyl, hydroxycarbonyl, methoxycarbonyl, ethoxycarbonyl, C₁-C₂-alkylaminocarbonyl, piperazinylcarbonyl, piperidinylcarbonyl or morpholinylcarbonyl, where piperazinylcarbonyl, piperidinylcarbonyl and morpholinylcarbonyl may each be substituted by 1 to 3 substituents, where the substituents are each independently selected from the group consisting of oxo, halogen and C₁-C₄-alkyl, and
where alkylaminocarbonyl may be substituted by one substituent, where the substituent is selected from the group consisting of piperazinyl, piperidinyl and morpholinyl,
where piperazinyl, piperidinyl and morpholinyl may each be substituted by 1 to 2 substituents, where the substituents are each independently selected from the group consisting of oxo, halogen and C₁-C₄-alkyl,
or
R⁴ is trifluoromethyl,
and
R⁵ is cyano,
or one of the salts thereof, solvates thereof or solvates of the salts thereof.

4. Compound according to any of Claims 1, 2 and 3, **characterized in that**
R¹ is a group of the formula 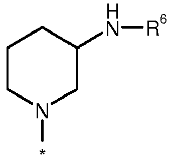 where
* is the attachment site to the heterocycle,
R⁶ is a group of the formula 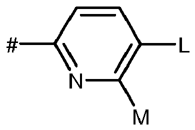 where
# is the attachment site to NH, either
L is cyano,
and
M is hydrogen,
or
L is cyano, nitro or trifluoromethylcarbonyl,
and
M is amino,
R² is phenyl,
where phenyl is substituted by 1 or 2 substituents, where the substituents are each independently selected from the group consisting of chlorine, fluorine and methoxy,
R³ is hydrogen,
either
R⁴ is hydrogen,
and
R⁵ is cyano, hydroxycarbonyl, methoxycarbonyl, methylaminocarbonyl, dimethylaminocarbonyl, piperazinylcarbonyl or piperidinylcarbonyl, where piperazinylcarbonyl and piperidinylcarbonyl are each substituted by 1 to 2 substituents, where the substituents are each independently selected from the group consisting of halogen and C₁-C₄-alkyl,
and
where methylaminocarbonyl and dimethylaminocarbonyl are substituted by one piperidinyl substituent,
in which piperidinyl is substituted by 1 to 2 substituents, where the substituents are each independently selected from the group consisting of halogen and C₁-C₄-alkyl,
or
R⁴ is trifluoromethyl,
and
R⁵ is cyano,
or one of the salts thereof, solvates thereof or solvates of the salts thereof.

5. Process for preparing a compound of the formula (I) or one of the salts thereof, solvates thereof or solvates of the salts thereof according to Claim 1, **characterized in that** either
[A] a compound of the formula 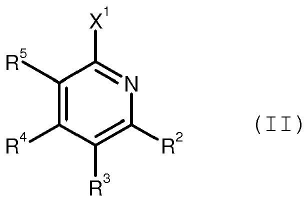 in which
R², R³, R⁴ and R⁵ are each as defined in Claim 1, and
X¹ is halogen, preferably chlorine or fluorine, is reacted with a compound of the formula
R¹-H (III)
in which
R¹ is as defined in Claim 1
or
[B] a compound of the formula 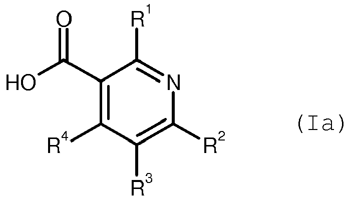 in which
R¹, R², R³ and R⁴ are each as defined in Claim 1, is reacted with a compound of the formula 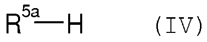 in which
R^{5a} is C₁-C₄-alkoxy, C₁-C₄-alkylamino or 5- to 6-membered heterocyclyl,
where heterocyclyl may be substituted by 1 to 3 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
and
where alkylamino may be substituted by one substituent, where the substituent is selected from the group consisting of hydroxyl, amino, hydroxycarbonyl, aminocarbonyl, C₁-C₄-alkoxy, C₁-C₄-alkylamino and 5- or 6-membered heterocyclyl,
in which heterocyclyl may be substituted by 1 to 2 substituents, where the substituents are each independently selected from the group consisting of oxo, halogen, C₁-C₄-alkyl, C₁-C₄-alkoxy, C₁-C₄-alkylamino, C₁-C₄-alkylcarbonyl, C₁-C₄-alkoxycarbonyl and C₁-C₄-alkylaminocarbonyl,
to give a compound of the formula

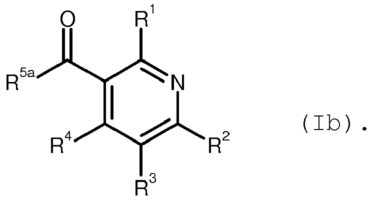

6. Compound according to any of Claims 1 to 4 for treatment and/or prophylaxis of diseases.

7. Use of a compound according to any of Claims 1 to 4 for producing a medicament for treatment and/or prophylaxis of diseases.

8. Use of a compound according to any of Claims 1 to 4 for producing a medicament for treatment and/or prophylaxis of haematological disorders.

9. Medicament comprising a compound according to any of Claims 1 to 4 in combination with an inert, nontoxic, pharmaceutically suitable excipient.

10. Medicament according to Claim 9 for treatment and/or prophylaxis of haematological disorders.

## Revendications

1. Composé de formule 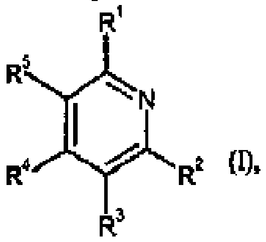 dans laquelle
R¹ représente un groupe de formule 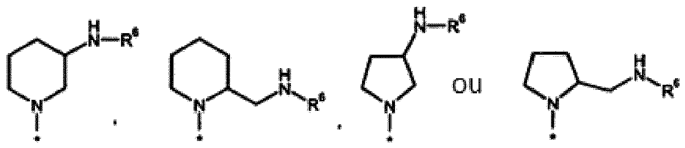 dans lesquelles
* signifie le site de liaison à l'hétérocycle,
R⁶ représente pyrid-2-yle, pyrimid-2-yle, 2-aminopyrimid-4-yle, 2-cyclopropylaminopyrimid-4-yle, 2-méthylaminopyrimid-4-yle, 2-éthylaminopyrimid-4-yle, 1,3-thiazol-2-yle, 1,3-thiazol-4-yle ou 1,2-pyrazol-5-yle, pyrid-2-yle, pyrimid-2-yle, 1,3-thiazol-2-yle et 1,3-thiazol-4-yle étant substitués par 1 à 2 substituants, les substituants étant choisis, indépendamment l'un de l'autre, dans le groupe constitué par halogène, cyano, nitro, amino, trifluorométhyle, trifluorométhoxy, aminocarbonyle, trifluorométhylcarbonyle, C₁-C₄-alkyle, C₁-C₄-alcoxy, C₁-C₄-alkylamino, C₁-C₄-alkylcarbonyle, C₁-C₄-alcoxycarbonyle, C₁-C₄-alkylaminocarbonyle et C₃-C₆-cycloalkylcarbonyle,
dans lesquels alkyle, alkylamino, alkylcarbonyle, alkylaminocarbonyle et cycloalkylcarbonyle peuvent être substitués par un substituant, le substituant étant choisi dans le groupe constitué par halogène, cyano, hydroxy, amino, trifluorométhyle et C₃-C₆-cycloalkyle, et
2-aminopyrimid-4-yle, 2-cyclopropylaminopyrimid-4-yle, 2-méthylaminopyrimid-4-yle, 2-éthylaminopyrimid-4-yle et 1,2-pyrazol-5-yle pouvant être substitués par 1 à 2 substituants, les substituants étant choisis, indépendamment l'un de l'autre, dans le groupe constitué par halogène, cyano, nitro, amino, trifluorométhyle, trifluorométhoxy, aminocarbonyle, trifluorométhylcarbonyle, C₁-C₄-alkyle, C₁-C₄-alcoxy, C₁-C₄-alkylamino, C₁-C₄-alkylcarbonyle, C₁-C₄-alcoxycarbonyle, C₁-C₄-alkylaminocarbonyle et C₃-C₆-cycloalkylcarbonyle,
R² représente phényle,
phényle pouvant être substitué par 1 à 3 substituants, les substituants étant choisis, indépendamment les uns des autres, dans le groupe constitué par hydroxy, halogène, cyano, trifluorométhyle, trifluorométhoxy, aminocarbonyle, C₁-C₄-alkyle, C₁-C₄-alcoxy, C₁-C₄-alkylamino, C₁-C₄-alcoxyméthyle, C₁-C₄-alkylaminométhyle, C₁-C₄-alkylcarbonyle, C₁-C₄-alcoxycarbonyle, C₁-C₄-alkylaminocarbonyle, C₁-C₄-alkylcarbonylamino, C₁-C₄-alkylsulfonyle, C₁-C₄-alkylsulfonylamino, C₁-C₄-alkylaminosulfonyle, phényle, pyrrolidinyle, pipéridinyle, morpholinyle, pipérazinyle, pyrrolidinylméthyle, pipéridinylméthyle, morpholinylméthyle et pipérazinylméthyle,
dans lesquels phényle, pyrrolidinyle, pipéridinyle, morpholinyle, pipérazinyle, pyrrolidinylméthyle, pipéridinylméthyle, morpholinylméthyle et pipérazinylméthyle peuvent être substitués par 1 à 3 substituants, les substituants étant choisis, indépendamment les uns des autres, dans le groupe constitué par halogène, cyano, trifluorométhyle, trifluorométhoxy et C₁-C₄-alkyle,
R³ représente hydrogène, halogène, cyano, trifluorométhyle, C₁-C₃-alkyle ou cyclopropyle,
soit
R⁴ représente hydrogène, halogène, cyano, trifluorométhyle, C₁-C₃-alkyle ou cyclopropyle, et
R⁵ représente halogène, cyano, trifluorométhyle, hydroxycarbonyle, aminocarbonyle, C₁-C₄-alkyle, C₁-C₄-alcoxycarbonyle, C₁-C₄-alkylaminocarbonyle ou hétérocyclylcarbonyle de 5 ou 6 chaînons, hétérocyclylcarbonyle pouvant être substitué par 1 à 3 substituants, les substituants étant choisis, indépendamment les uns des autres, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, et alkylaminocarbonyle pouvant être substitué par un substituant, le substituant étant choisi dans le groupe constitué par hydroxy, amino, hydroxycarbonyle, aminocarbonyle, C₁-C₄-alcoxy, C₁-C₄-alkylamino et hétérocyclyle de 5 ou 6 chaînons, hétérocyclyle pouvant être substitué par 1 à 2 substituants, les substituants étant choisis, indépendamment l'un de l'autre, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, soit
R⁴ représente halogène, cyano, trifluorométhyle, hydroxycarbonyle, aminocarbonyle, C₁-C₄-alkyle, C₁-C₄-alcoxycarbonyle, C₁-C₄-alkylaminocarbonyle ou hétérocyclylcarbonyle de 5 ou 6 chaînons, hétérocyclylcarbonyle pouvant être substitué par 1 à 3 substituants, les substituants étant choisis, indépendamment les uns des autres, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, et alkylaminocarbonyle pouvant être substitué par un substituant, le substituant étant choisi dans le groupe constitué par hydroxy, amino, hydroxycarbonyle, aminocarbonyle, C₁-C₄-alcoxy, C₁-C₄-alkylamino et hétérocyclyle de 5 ou 6 chaînons, hétérocyclyle pouvant être substitué par 1 à 2 substituants, les substituants étant choisis, indépendamment l'un de l'autre, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, et
R⁵ représente hydrogène, halogène, cyano, trifluorométhyle, C₁-C₃-alkyle ou cyclopropyle,
ou un de ses sels, de ses solvates ou des solvates de ses sels.

2. Composé selon la revendication 1, **caractérisé en ce que**
R¹ représente un groupe de formule 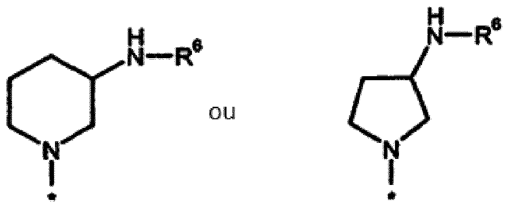 dans lesquelles
* signifie le site de liaison à l'hétérocycle,
R⁶ représente pyrid-2-yle, pyrimid-2-yle, 2-aminopyrimid-4-yle, 2-cyclopropylaminopyrimid-4-yle, 2-méthylaminopyrimid-4-yle, 2-éthylaminopyrimid-4-yle, 1,3-thiazol-2-yle, 1,3-thiazol-4-yle ou 1,2-pyrazol-5-yle, pyrid-2-yle, pyrimid-2-yle, 1,3-thiazol-2-yle et 1,3-thiazol-4-yle étant substitués par 1 à 2 substituants, les substituants étant choisis, indépendamment l'un de l'autre, dans le groupe constitué par halogène, cyano, nitro, amino, trifluorométhyle, trifluorométhoxy, aminocarbonyle, trifluorométhylcarbonyle, C₁-C₄-alkyle, C₁-C₄-alcoxy, C₁-C₄-alkylamino, C₁-C₄-alkylcarbonyle, C₁-C₄-alcoxycarbonyle, C₁-C₄-alkylaminocarbonyle et C₃-C₆-cycloalkylcarbonyle,
dans lesquels alkyle, alkylamino, alkylcarbonyle, alkylaminocarbonyle et cycloalkylcarbonyle peuvent être substitués par un substituant, le substituant étant choisi dans le groupe constitué par halogène, cyano, hydroxy, amino, trifluorométhyle et C₃-C₆-cycloalkyle, et 2-aminopyrimid-4-yle, 2-cyclopropylaminopyrimid-4-yle, 2-méthylaminopyrimid-4-yle, 2-éthylaminopyrimid-4-yle et 1,2-pyrazol-5-yle pouvant être substitués par 1 à 2 substituants, les substituants étant choisis, indépendamment l'un de l'autre, dans le groupe constitué par halogène, cyano, nitro, amino, trifluorométhyle, trifluorométhoxy, aminocarbonyle, trifluorométhylcarbonyle, C₁-C₄-alkyle, C₁-C₄-alcoxy, C₁-C₄-alkylamino, C₁-C₄-alkylcarbonyle, C₁-C₄-alcoxycarbonyle, C₁-C₄-alkylaminocarbonyle et C₃-C₆-cycloalkylcarbonyle,
R² représente phényle,
phényle pouvant être substitué par 1 à 3 substituants, les substituants étant choisis indépendamment les uns des autres dans le groupe constitué par chlore, fluor, trifluorométhyle, trifluorométhoxy, méthyle et méthoxy
R³ représente hydrogène,
soit
R⁴ représente hydrogène et
R⁵ représente halogène, cyano, trifluorométhyle, hydroxycarbonyle, aminocarbonyle, C₁-C₄-alkyle, C₁-C₄-alcoxycarbonyle, C₁-C₄-alkylaminocarbonyle ou hétérocyclylcarbonyle de 5 ou 6 chaînons, hétérocyclylcarbonyle pouvant être substitué par 1 à 3 substituants, les substituants étant choisis, indépendamment les uns des autres, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, et alkylaminocarbonyle pouvant être substitué par un substituant, le substituant étant choisi dans le groupe constitué par hydroxy, amino, hydroxycarbonyle, aminocarbonyle, C₁-C₄-alcoxy, C₁-C₄-alkylamino et hétérocyclyle de 5 ou 6 chaînons, hétérocyclyle pouvant être substitué par 1 à 2 substituants, les substituants étant choisis, indépendamment l'un de l'autre, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle,
soit
R⁴ représente trifluorométhyle et
R⁵ représente cyano,
ou un de ses sels, de ses solvates ou des solvates de ses sels.

3. Composé selon l'une quelconque des revendications 1 ou 2, **caractérisé en ce que**
R¹ représente un groupe de formule 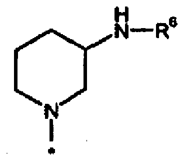 dans laquelle
* signifie le site de liaison à l'hétérocycle,
R⁶ représente pyrid-2-yle ou 1,3-thiazol-2-yle, pyrid-2-yle et 1,3-thiazol-2-yle étant substitué par 1 à 2 substituants, les substituants étant choisis, indépendamment l'un de l'autre, dans le groupe constitué par cyano, nitro, amino, trifluorométhylcarbonyle, éthylcarbonyle et méthylcarbonyle,
R² représente phényle,
phényle étant substitué par 1 à 2 substituants, les substituants étant choisis indépendamment l'un de l'autre dans le groupe constitué par chlore, fluor, trifluorométhyle, trifluorométhoxy, méthyle et méthoxy,
R³ représente hydrogène,
soit
R⁴ représente hydrogène et
R⁵ représente cyano, trifluorométhyle, hydroxycarbonyle, méthoxycarbonyle, éthoxycarbonyle, C₁-C₂-alkylaminocarbonyle, pipérazinylcarbonyle, pipéridinylcarbonyle ou morpholinylcarbonyle, pipérazinylcarbonyle, pipéridinylcarbonyle et morpholinylcarbonyle pouvant être substitués par 1 à 3 substituants, les substituants étant choisis, indépendamment les uns des autres, dans le groupe constitué par oxo, halogène et C₁-C₄-alkyle, et alkylaminocarbonyle pouvant être substitué par un substituant, le substituant étant choisi dans le groupe constitué par pipérazinyle, pipéridinyle et morpholinyle,
pipérazinyle, pipéridinyle et morpholinyle pouvant être substitués par 1 à 2 substituants, les substituants étant choisis, indépendamment l'un de l'autre, dans le groupe constitué par oxo, halogène et C₁-C₄-alkyle, ou
R⁴ représente trifluorométhyle et
R⁵ représente cyano,
ou un de ses sels, de ses solvates ou des solvates de ses sels.

4. Composé selon l'une quelconque des revendications 1, 2, ou 3, **caractérisé en ce que**
R¹ représente un groupe de formule 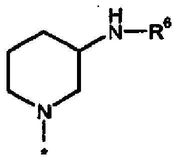 dans laquelle
* signifie le site de liaison à l'hétérocycle,
R⁶ représente un groupe de formule 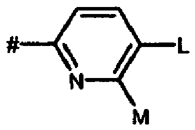 dans laquelle
# signifie le site de liaison à NH, soit
L représente cyano, et
M représente hydrogène,
soit
L représente cyano, nitro ou trifluorométhylcarbonyle, et
M représente amino,
R² représente phényle, phényle étant substitué par 1 à 2 substituants, les substituants étant choisis indépendamment l'un de l'autre dans le groupe constitué par chlore, fluor et méthoxy,
R³ représente hydrogène,
soit
R⁴ représente hydrogène et
R⁵ représente cyano, hydroxycarbonyle, méthoxycarbonyle, méthylaminocarbonyle, diméthylaminocarbonyle, pipérazinylcarbonyle ou pipéridinylcarbonyle, pipérazinylcarbonyle et pipéridinylcarbonyle étant substitués par 1 à 2 substituants, les substituants étant choisis, indépendamment l'un de l'autre, dans le groupe constitué par halogène et C₁-C₄-alkyle, et méthylaminocarbonyle et diméthylaminocarbonyle étant substitués par un substituant pipéridinyle, pipéridinyle étant substitué par 1 à 2 substituants, les substituants étant choisis, indépendamment l'un de l'autre, dans le groupe constitué par halogène et C₁-C₄-alkyle,
soit
R⁴ représente trifluorométhyle et
R⁵ représente cyano,
ou un de ses sels, de ses solvates ou des solvates de ses sels.

5. Procédé pour la préparation d'un composé de formule (I) ou d'un de ses sels, de ses solvates ou des solvates de ses sels selon la revendication 1, **caractérisé en ce qu'**on transforme
soit
[A] un composé de formule 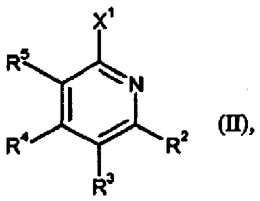 dans laquelle
R², R³, R⁴ et R⁵ ont la signification indiquée dans la revendication 1 et
X¹ représente halogène, de préférence chlore ou fluor,
avec un composé de formule
R¹-H (III),
dans laquelle
R¹ a la signification indiquée dans la revendication 1,
soit
[B] un composé de formule 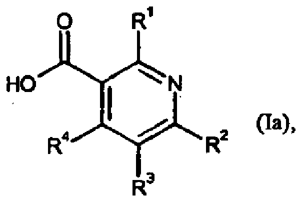 dans laquelle
R¹, R², R³ et R⁴ présentent la signification indiquée dans la revendication 1,
avec un composé de formule
R^{5a}-H (IV),
dans laquelle
R^{5a} représente C₁-C₄-alcoxy, C₁-C₄-alkylamino ou hétérocyclyle de 5 ou 6 chaînons, hétérocyclyle pouvant être substitué par 1 à 3 substituants, les substituants étant choisis, indépendamment les uns des autres, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle, et alkylamino pouvant être substitué par un substituant, le substituant étant choisi dans le groupe constitué par hydroxy, amino, hydroxycarbonyle, aminocarbonyle, C₁-C₄-alcoxy, C₁-C₄-alkylamino et hétérocyclyle de 5 ou 6 chaînons, hétérocyclyle pouvant être substitué par 1 à 2 substituants, les substituants étant choisis, indépendamment l'un de l'autre, dans le groupe constitué par oxo, halogène, C₁-C₄-alkyle, C₁-C₄-alcoxy, C₁-C₄-alkylamino, C₁-C₄-alkylcarbonyle, C₁-C₄-alcoxycarbonyle et C₁-C₄-alkylaminocarbonyle,
en un composé de formule

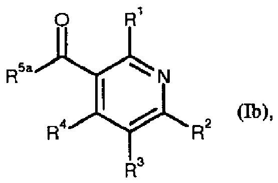

6. Composé selon l'une quelconque des revendications 1 à 4 destiné au traitement et/ou à la prophylaxie de maladies.

7. Utilisation d'un composé selon l'une quelconque des revendications 1 à 4 pour la préparation d'un médicament destiné au traitement et/ou à la prophylaxie de maladies.

8. Utilisation d'un composé selon l'une quelconque des revendications 1 à 4 pour la préparation d'un médicament destiné au traitement et/ou à la prophylaxie de maladies hématologiques.

9. Médicament contenant un composé selon l'une quelconque des revendications 1 à 4 en combinaison avec un adjuvant inerte, non toxique, pharmaceutiquement approprié.

10. Médicament selon la revendication 9 destiné au traitement et/ou à la prophylaxie de maladies hématologiques.
